# EUROPÄISCHE PATENTSCHRIFT

(11) **EP 1 778 627 B1**
(45) Veröffentlichungstag und Bekanntmachung des Hinweises auf die Patenterteilung: **27.02.2008**
(21) Anmeldenummer: 05758872.5
(22) Anmeldetag: 28.06.2005
(51) Int. Cl.: C07C 271/22, C07C 233/85, C07C 233/83, C07D 213/74, C07D 277/42, C07C 231/18, C07C 269/06

(54) **VERFAHREN ZUR HERSTELLUNG DER ENANTIOMEREN FORMEN VON 2,3-DIAMINOPROPIONSÄUREDERIVATEN**
METHOD FOR PRODUCING ENANTIOMERIC FORM OF 2, 3-DIAMINOPROPIONIC ACID DERIVATIVES
PROCEDE DE PRODUCTION DE FORMES ENANTIOMERES DE DERIVES DE L'ACIDE 2,3-DIAMINOPROPIONIQUE

(30) Priorität: 10.07.2004 DE 102004033406
(43) Veröffentlichungstag der Anmeldung: 02.05.2007
(73) Patentinhaber: Sanofi-Aventis Deutschland GmbH, 65929 Frankfurt am Main (DE)
(72) Erfinder: RIEKE-ZAPP, Joerg, 60325 Frankfurt (DE); BILLEN, Guenter, 65527 Niedernhausen (DE)
(86) Internationale Anmeldenummer: PCT/EP2005/006920
(87) Internationale Veröffentlichungsnummer: WO 2006/005436

(56) Entgegenhaltungen:
- WO-A-20/04022553
- A.J.ROBINSON: "Highly enantioselective synthesis of alpha,beta-diaminopropanoic acid derivatives using a catalytic asymmetric hydrogenation approach" J.ORG.CHEM., Bd. 66, Nr. 12, 2001, Seiten 4141-4147, XP002353243 in der Anmeldung erwähnt

## Beschreibung

Die Erfindung betrifft ein Verfahren zur Herstellung der enantiomeren Formen von 2,3-Diaminopropionsäurederivaten der Formel I durch asymmetrische Hydrierung. Die Verbindungen der Formel I sind geeignete Zwischenprodukte zur Herstellung von IkB-Kinase Inhibitoren (WO 01/30774 A1; WO2004/022553).

Es ist bekannt, dass man α,β-Diaminopropionsäurederivate durch Rh-katalysierte asymmetrische Hydrogenierung entsprechend Schema 1 herstellen kann (J Org Chem, Vol. 66, 11, 2001, Seiten 4141-4147). Die asymmetrische Hydrierung gelingt jedoch nur, wenn beide N-Atome acyliert sind. 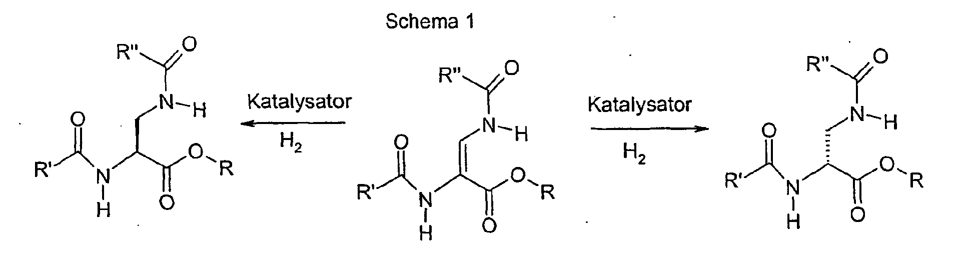 Der Versuch N,N-Dimethylen-amine oder N,N-Dimethylen-enamine zu hydrieren war erfolglos.

Es wurde nun gefunden, dass die asymmetrische Synthese auch bei Verbindungen der Formel II gelingt. Die Synthese der Verbindung der Formel I gelingt mit hohen Ausbeuten und hoher Enantioselektivität.

Die Erfindung betrifft daher ein Verfahren zur Gewinnung der Verbindung der Formel I, 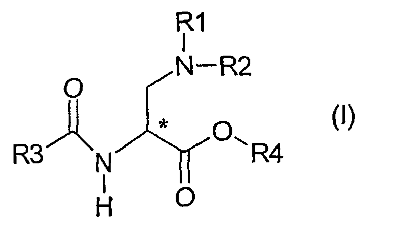 wobei R1 für
1) Wasserstoffatom,
2) -(C₁-C₄)-Alkyl,
3) -(C₆-C₁₄)-Aryl, wobei Aryl unsubstituiert oder unabhängig voneinander ein-, zwei- oder dreifach durch R11 substituiert ist, wobei R11 für
   a) F, CI, J oder Br,
   b) -(C₁-C₄)-Alkyl,
   c) -CN,
   d) -CF₃,
   e) -OR⁵, worin R⁵ für Wasserstoffatom oder -(C₁-C₄)-Alkyl steht,
   f) -N(R⁵)-R⁶, worin R⁵ und R⁶ unabhängig voneinander für Wasserstoffatom oder -(C₁-C₄)-Alkyl stehen,
   g) -C(O)-R⁵, worin R⁵ für Wasserstoffatom oder -(C₁-C₄)-Alkyl steht,
      oder
   h) -S(O)ₓ-R⁵, worin x die ganze Zahl Null, 1 oder 2 bedeutet und R⁵ Wasserstoffatom oder -(C₁-C₄)-Alkyl bedeutet, steht,
4) -CH(R7)-Aryl, worin Aryl unsubstituiert oder unabhängig voneinander
   ein-, zwei- oder dreifach durch -NO₂, -O-CH₃, F, Cl oder Brom substituiert ist, worin R7 Wasserstoffatom oder -(C₁-C₄)-Alkyl bedeutet, oder
5) 4- bis 15-gliedriger Het-Ring, stehen, wobei der Het-Ring unsubstituiert ist oder ein-, zwei- oder dreifach unabhängig voneinander substituiert ist durch -(C₁-C₅)-Alkyl, -(C₁-C₅)-Alkoxy, Halogen, Nitro, Amino, Trifluormethyl, Hydroxyl, Hydroxy-(C₁-C₄)-Alkyl, Methylendioxy, Ethylendioxy, Formyl, Acetyl, Cyano, Hydroxycarbonyl, Aminocarbonyl oder -(C₁-C₄)-Alkoxycarbonyl,
R2 für
1) -(C₆-C₁₄)-Aryl, wobei Aryl unsubstituiert oder unabhängig voneinander ein-, zwei- oder dreifach durch R11 substituiert ist,
2) -CH(R7)-Aryl, worin Aryl unsubstituiert oder unabhängig voneinander ein-zwei- oder dreifach durch -NO₂, -O-CH₃, F, Cl oder Brom substituiert ist, worin R7 Wasserstoffatom oder -(C₁-C₄)-Alkyl bedeutet, oder
3) 4- bis 15-gliedriger Het-Ring, steht, wobei der Het-Ring unsubstituiert ist oder ein-, zwei- oder dreifach unabhängig voneinander substituiert ist durch -(C₁-C₅)-Alkyl, -(C₁-C₅)-Alkoxy, Halogen, Nitro, Amino, Trifluormethyl, Hydroxyl, Hydroxy-(C₁-C₄)-Alkyl, Methylendioxy, Ethylendioxy, Formyl, Acetyl, Cyano, Hydroxycarbonyl, Aminocarbonyl oder -(C₁-C₄)-Alkoxycarbonyl,
R3 für
1) Wasserstoffatom,
2) -(C₁-C₄)-Alkyl,
3) -(C₆-C₁₄)-Aryl, worin Aryl unsubstituiert oder unabhängig voneinander
   ein-, zwei- oder dreifach durch -NO₂, -O-(C₁-C₄)-Alkyl, F, Cl oder Brom substituiert ist,
4) -O-C(CH₃)₃, oder
5) -O-CH(R7)-Aryl, worin Aryl unsubstituiert oder unabhängig voneinander
   ein-, zwei- oder dreifach durch -NO₂, -O-CH₃, F, Cl oder Brom substituiert ist,
   worin R7 Wasserstoffatom oder -(C₁-C₄)-Alkyl bedeutet,
R4 für
1) Wasserstoffatom,
2) -(C₁-C₄)-Alkyl oder
3) -CH(R8)-Aryl steht,
   worin R8 Wasserstoffatom oder -(C₁-C₄)-Alkyl bedeutet,
das dadurch gekennzeichnet ist, dass man
eine Verbindung der Formel II 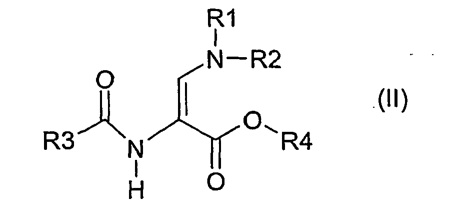 worin R1, R2, R3 und R4 die gleiche Bedeutung wie in der Formel I haben und die
Verbindung in der E als auch der Z Konfiguration an der Doppelbindung vorliegen kann,
in Anwesenheit von Wasserstoff und einem Katalysator hydriert.

Die Erfindung betrifft ferner ein Verfahren zur Gewinnung der Verbindung der Formel I, wobei R1 für Phenyl oder Wasserstoffatom steht,
R2 für Phenyl, Pyridyl oder Thiazolyl steht, worin Phenyl, Pyridyl oder Thiazolyl unsubstituiert oder durch Fluor oder Chlor substituiert sind, und
R3 für Phenyl oder -O-CH₂-Phenyl steht und
R4 für Methyl oder Ethyl steht.

Die Erfindung betrifft ferner ein Verfahren zur Gewinnung der Verbindung der Formel III, 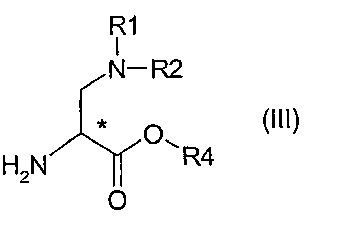 oder deren Salze, worin R1, R2 und R4 die gleiche Bedeutung wie in der Formel I haben,
das dadurch gekennzeichnet ist, dass man
a) die Verbindung der Formel II, 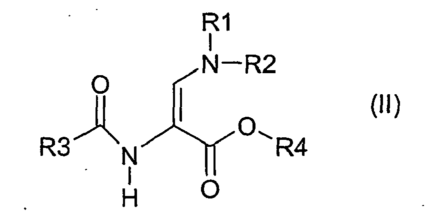 worin R1, R2, R3 und R4 die gleiche Bedeutung wie in der Formel I haben,
   in Anwesenheit von Wasserstoff und einem Katalysator hydriert und in eine Verbindung der Formel I 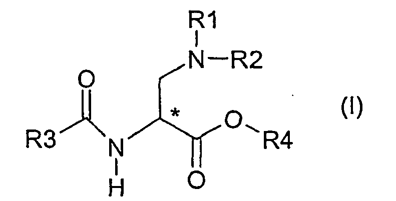 umsetzt, und
b) die erhaltene Verbindung der Formel I in eine Verbindung der Formel III überführt.

Der Verfahrensschritt b) wird beispielsweise gemäß den Reaktionsbedingungen wie sie von T. Greene, P. Wuts in Protective Groups in Organic Synthesis, Wiley-Interscience, für die Spaltung von Amiden oder Carbamaten beschrieben sind, durchgeführt. Die direkte Überführung von Verbindungen der Formel I in Verbindungen der Formel III führt, je nach gewählten Reaktionsbedingungen, hier insbesondere stark basische Reaktionsbedingungen, zu einer Racemisierung des durch die asymmetrische Hydrierung aufgebauten, chiralen Zentrums oder zu anderen unerwünschten Nebenreaktionen. Dies kann vermieden werden, wenn die Verbindung der Formel I mit beispielsweise *tert.*-Butyldicarbonat oder einem anderen Reagenz zur Einführung von *tert.*-Butoxycarbonyl-Schutzgruppen in die Verbindung IV überführt wird. Die Einführung der *tert.*-Butoxycarbonyl-Schutzgruppe erfolgt in einem geeigneten Lösungsmittel wie Acetonitril, Tetrahydrofuran oder Toluol bevorzugt mit Hilfe eines Acylierungskatalysators wie N,N-Dimethylaminopyridin (DMAP). Die Reaktionstemperatur beträgt dabei von 0 °C bis 120 °C, bevorzugt von 20 °C bis 40 °C.
Die Reaktionszeit liegt im allgemeinen von 0,5 bis 24 Stunden, je nach Zusammensetzung des Gemisches und des gewählten Temperaturbereiches.
Die erhaltene Verbindung der Formel IV wird anschließend unter milden Bedingungen, wie Magnesiummethylat, in eine Verbindung der Formel Ia überführt.
Die Umwandlung in die Verbindungen der Formel III geschieht üblicherweise unter literaturbekannten Reaktionsbedingungen, wie sie von T. Greene, P. Wuts in Protective Groups in Organic Synthesis, Wiley-Interscience, für die Spaltung von tert.-Butyloxycarbonyl (BOC) Schutzgruppen beschrieben sind.

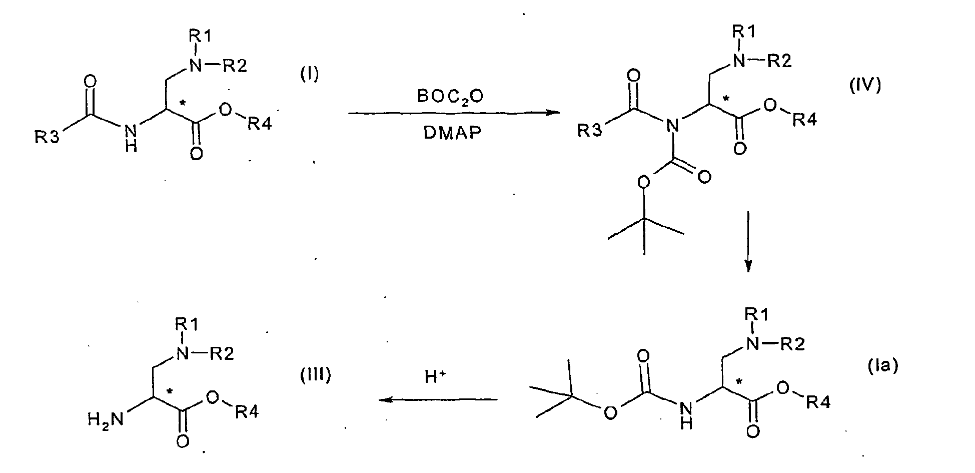

Die Erfindung betrifft daher ferner ein Verfahren zur Gewinnung der Verbindung der Formel III,
das dadurch gekennzeichnet ist, dass man
a) die Verbindung der Formel II,
   worin R1, R2, R3 und R4 die gleiche Bedeutung wie in der Formel I haben,
   in Anwesenheit von Wasserstoff und einem Katalysator hydriert und in eine Verbindung der Formel I umsetzt,
b) die erhaltene Verbindung der Formel I mit einem *tert*.-Butyldicarbonat und einem Acylierungskatalysator wie Dimethylaminopyridin (DMAP), in eine Verbindung der Formel IV umsetzt, 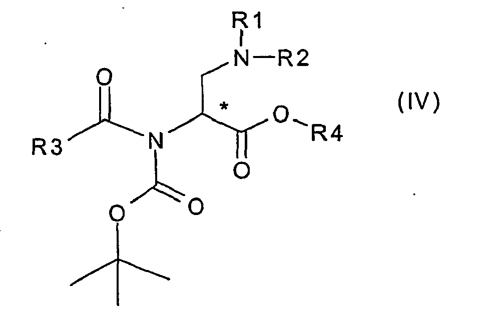 worin R1, R2, R3 und R4 die Bedeutung wie in Formel I haben,
c) die erhaltene Verbindung der Formel IV anschließend in die Verbindung der Formel Ia 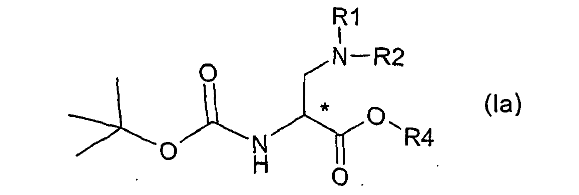 überführt, worin R1, R2, R3 und R4 die Bedeutung wie in Formel I haben, und
d) die erhaltene Verbindung der Formel Ia in die Verbindung der Formel III oder deren Salze, überführt, worin R1, R2, und R4 die Bedeutung wie in Formel I haben.

Die Überführung der Verbindungen der Formel IV in die Verbindung der Formel Ia wird beispielsweise durch Behandlung mit Basen, wie Lithiumhydroxid, Hydrazin oder Magnesiummethylat erreicht (Literatur: J.Org.Chem. 1997, 62, 7054-7057). Die Abspaltung der tert-Butyloxycarbonylgruppe zu Verbindungen der Formel III erfolgt unter Standardbedingungen, wie die Behandlung mit Trifluoressigsäure (TFA), Salzsäure oder p-Toluolsulfonsäure in geeigneten Lösungsmitteln.
Die Abreicherung des unerwünschten Enantiomers erfolgt durch Kristallisation der Verbindungen der Formel I oder III aus geeigneten Lösungsmitteln wie Methanol, Ethanol, 1-Propanol, 2-Propanol, n-Butanol, 2-Butanol und deren Ester. Bei Verbindungen der Formel III wird die Kristallisation bevorzugt in Form ihrer (sauren) Salze, wie Hydrochlorid, Methansulfonat oder p-Toluolsulfonat durchgeführt. Unter diesen Bedingungen werden optische Reinheiten von > 99% erreicht. Zweckmäßigerweise kann die gesamte Reaktionssequenz ohne Isolierung der Verbindungen IV und Ia in einem Eintopf-Verfahren durchgeführt werden. Die hierbei erzielten Ausbeuten und optischen Reinheiten entsprechen den oben genannten Werten.

Unter dem Begriff "Katalysator" werden Verbindungen verstanden, wie sie beispielsweise von E. N. Jacobson, A. Pfaltz, H. Yamamoto in Comprehensive Asymmetric Catalysis, Springer-Verlag, 1999 oder X. Zhang, Chemical Reviews, 2003, 103, 3029-3069 und der dort zitierten Literatur beschrieben werden, beispielsweise optisch aktive Rhodium-, Ruthenium- oder Iridiumkomplexe oder Gemische davon. Der katalytisch wirksame Komplex wird dabei durch Reaktion eines Metallkomplexes mit einem optisch aktiven Phosphin gebildet. Bei den oben beschriebenen acylierten 2,3-Diaminopropionsäurederivaten zeigten die Me-Duphos oder Et-Duphos Rhodium Komplexe sehr gute Enantioselektivitäten und Umsätze. Es ist weiterhin bekannt, dass zur Herstellung chiraler β-Aminosäuren Rhodium Komplexe von Typ BICP, t-Bu-BisP, BDPMI, Et-FerroTANE, MaIPHOS und MonoPHOS als Katalysatoren eingesetzt werden können.

Unter den Begriffen "-(C₁-C₄)-Alkyl" oder "(C₁-C₅)-Alkyl" werden Kohlenwasserstoffreste verstanden, deren Kohlenstoffkette geradkettig oder verzweigt ist und 1 bis 4 oder 1 bis 5 Kohlenstoffatome enthält, beispielsweise Methyl, Ethyl, Propyl, Iso-Propyl, Butyl, Iso-Butyl, tertiär-Butyl oder Pentyl.

Unter den Begriffen "CH(R7)-" oder "CH(R8)-" werden geradkettige oder verzweigte Kohlenwasserstoffreste verstanden, wie Methylen, Ethylen, Isopropylen, Isobutylen oder Pentylen. Der Rest "-CH(R7)-Aryl" ist beispielsweise für den Fall, dass R7 Wasserstoffatom und Aryl Phenyl ist, der Rest Benzyl.

Unter den Begriffen "-(C₆-C₁₄-Ary)," oder "Aryl" werden aromatische Kohlenstoffreste verstanden mit 6 bis 14 Kohlenstoffatomen im Ring. -(C₆-C₁₄)-Arylreste sind beispielsweise Phenyl, Naphthyl, zum Beispiel 1-Naphthyl, 2-Naphthyl, Anthryl oder Fluorenyl. Naphthylreste und insbesondere Phenylreste sind bevorzugte Arylreste.

Unter dem Begriff "4- bis 15-gliedriger Het-Ring" werden Ringsysteme verstanden mit 4 bis 15 Kohlenstoffatomen, die in ein, zwei oder drei miteinander verbundenen Ringsystemen vorliegen und die ein, zwei, drei oder vier gleiche oder verschiedene Heteroatome aus der Reihe Sauerstoff, Stickstoff oder Schwefel enthalten. Beispiele für diese Ringsysteme sind die Reste Acridinyl, Azepinyl, Azetidinyl, Aziridinyl, Benzimidazalinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl; 1H-Indazolyl, Indolinyl, Indolizinyl, Indolyl, 3H-Indolyl, Isobenzofuranyl, lsochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl und Xanthenyl.

Der Stern an einem Kohlenstoffatom in der Verbindung der Formel I oder II bedeutet, dass das jeweilige Kohlenstoffatom chiral ist und die Verbindung entweder als R- oder S-Enantiomer vorliegt.

Die asymmetrische Hydrierung der Verbindungen gemäß Formel II wird vorteilhaft bei einer Temperatur von 10 °C bis 200 °C und einem Wasserstoffdruck von 1 bar bis 200 bar durchgeführt. Das molare Verhältnis Katalysator-Edukt liegt vorteilhaft bei 1:100 bis 1:10000. Als Lösungsmittel für die asymmetrische Hydrierung eignen sich beispielsweise Wasser, niedrige Alkohole wie Methanol, Ethanol, Propanol oder Isopropanol, aromatische Kohlenwasserstoffe wie Toluol, Ketone wie Aceton, halogenierte Kohlenwasserstoffe wie Dichlormethan, Carbonsäureester wie Ethylacetat und Ether wie Tetrahydrofuran.

Die optisch aktiven 2,3-Diaminopropionsäurederivate der Formel I, III und IV sind als solche, auch in Form ihrer Enantiomerengemische und ihrer Salze, ebenfalls Gegenstand der vorliegenden Erfindung. Unter Enantiomerengemischen sind hier insbesondere solche zu verstehen, in welchen ein Enantiomer gegenüber dem anderen angereichert ist.

Die Verbindungen der Formel II sind entweder bekannt oder lassen sich beispielsweise herstellen indem man Verbindungen der Formel IV worin R3 und R4 die oben genannte Bedeutung haben, mit einem Amin der Formel V, worin R1 und R2 die oben genannten Bedeutungen haben, umsetzt. 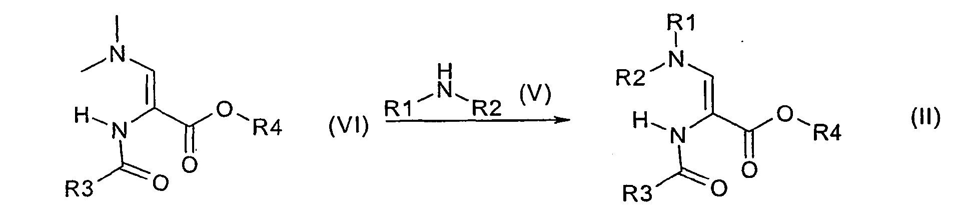 Die Reaktionstemperatur beträgt dabei von 0 °C bis 120 °C, bevorzugt von 20 °C bis 60 °C.

Die Reaktionszeit liegt im allgemeinen von 0,5 bis 8 Stunden, je nach Zusammensetzung des Gemisches und des gewählten Temperaturbereiches.
Die erhaltene Verbindung der Formel II wird anschließend durch wässrige Aufarbeitung und Extraktion mit einem geeigneten Lösungsmittel, beispielsweise Ethylacetat oder Dichlormethan oder durch Kristallisation aus dem Reaktionsgemisch abgetrennt.

Ein weiterer Aspekt der Erfindung betrifft neue Verbindungen der Formel II, wobei
R1 und R2 gleich oder verschieden sind und unabhängig voneinander für
1) -(C₆-C₁₄)-Aryl, wobei Aryl unsubstituiert oder unabhängig voneinander ein-, zwei- oder dreifach durch R11 substituiert ist, wobei R11 für
   a) F, Cl, J oder Br,
   b) -(C₁-C₄)-Alkyl,
   c) -CN,
   d) -CF₃,
   e) -OR⁵, worin R⁵ für Wasserstoffatom oder -(C₁-C₄)-Alkyl steht,
   f) -N(R⁵)-R⁶, worin R⁵ und R⁶ unabhängig voneinander für Wasserstoffatom oder -(C₁-C₄)-Alkyl stehen,
   g) -C(O)-R⁵, worin R⁵ für Wasserstoffatom oder -(C₁-C₄)-Alkyl steht, oder
   h) -S(O)ₓ-R⁵, worin x die ganze Zahl Null, 1 oder 2 bedeutet und R⁵ Wasserstoffatom oder -(C₁-C₄)-Alkyl bedeutet, steht, oder
2) 4- bis 15-gliedriger Het-Ring, stehen, wobei der Het-Ring unsubstituiert ist oder ein-, zwei- oder dreifach unabhängig voneinander substituiert ist durch
   -(C₁-C₅)-Alkyl, -(C₁-C₅)-Alkoxy, Halogen, Nitro, Amino, Trifluormethyl, Hydroxyl, Hydroxy-(C₁-C₄)-Alkyl, Methylendioxy, Ethylendioxy, Formyl, Acetyl, Cyano, Hydroxycarbonyl, Aminocarbonyl oder-(C₁-C₄)-Alkoxycarbonyl,
R3 für
1) -(C₆-C₁₄)-Aryl, worin Aryl unsubstituiert oder unabhängig voneinander
   ein-, zwei- oder dreifach durch -NO₂, -O-(C₁-C₄)-Alkyl, F, Cl oder Brom substituiert ist,
2) -O-C(CH₃)₃, oder
3) -O-CH(R7)-Aryl, worin Aryl unsubstituiert oder unabhängig voneinander
   ein-, zwei- oder dreifach durch -NO₂, -O-CH₃, F, Cl oder Brom substituiert ist, worin R7 Wasserstoffatom oder -(C₁-C₄)-Alkyl bedeutet,
R4 für
1) Wasserstoffatom,
2) -(C₁-C₄)-Alkyl oder
3) -CH(R8)-Aryl steht,
worin R8 Wasserstoffatom oder -(C₁-C₄)-Alkyl bedeutet.

Ein weiterer Aspekt der Erfindung betrifft neue Verbindungen der Formel IV, 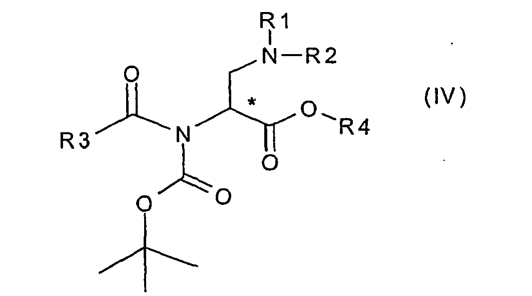 wobei R1 und R2 gleich oder verschieden sind und unabhängig voneinander für
1) -(C₆-C₁₄)-Aryl, wobei Aryl unsubstituiert oder unabhängig voneinander ein-, zwei- oder dreifach durch R11 substituiert ist, wobei R11 für
   a) F, Cl, J oder Br,
   b) -(C₁-C₄)-Alkyl,
   c) -CN,
   d) -CF₃,
   e) -OR⁵, worin R⁵ für Wasserstoffatom oder -(C₁-C₄)-Alkyl steht,
   f) -N(R⁵)-R⁶, worin R⁵ und R⁶ unabhängig voneinander für Wasserstoffatom oder -(C₁-C₄)-Alkyl stehen,
   g) -C(O)-R⁵, worin R⁵ für Wasserstoffatom oder -(C₁-C₄)-Alkyl steht, oder
   h) -S(O)ₓ-R⁵, worin x die ganze Zahl Null, 1 oder 2 bedeutet und R⁵ Wasserstoffatom oder -(C₁-C₄)-Alkyl bedeutet, steht, oder
2) 4- bis 15-gliedriger Het-Ring, stehen, wobei der Het-Ring unsubstituiert ist oder ein-, zwei- oder dreifach unabhängig voneinander substituiert ist durch
   -(C₁-C₅)-Alkyl, -(C₁-C₅)-Alkoxy, Halogen, Nitro, Amino, Trifluormethyl, Hydroxyl, Hydroxy-(C₁-C₄)-Alkyl, Methylendioxy, Ethylendioxy, Formyl, Acetyl, Cyano, Hydroxycarbonyl, Aminocarbonyl oder -(C₁-C₄)- Alkoxycarbonyl,
R3 für
1) -(C₆-C₁₄)-Aryl, worin Aryl unsubstituiert oder unabhängig voneinander
   ein-, zwei- oder dreifach durch -NO₂, -O-(C₁-C₄)-Alkyl, F, Cl oder Brom substituiert ist,
2) -O-C(CH₃)₃, oder
3) -O-CH(R7)-Aryl, worin Aryl unsubstituiert oder unabhängig voneinander
   ein-, zwei- oder dreifach durch -NO₂, -O-CH₃, F, Cl oder Brom substituiert ist,
   worin R7 Wasserstoffatom oder -(C₁-C₄)-Alkyl bedeutet,
R4 für
1) Wasserstoffatom,
2) -(C₁-C₄)-Alkyl oder
3) -CH(R8)-Aryl steht,
worin R8 Wasserstoffatom oder -(C₁-C₄)-Alkyl bedeutet.

Die Erfindung betrifft daher ferner ein Verfahren zur Gewinnung der neuen Verbindungen der Formel IV, das dadurch gekennzeichnet ist, dass man
a) die Verbindung der Formel II,
   worin R1, R2, R3 und R4 die gleiche Bedeutung wie in der neuen Verbindung der Formel II haben,
   in Anwesenheit von Wasserstoff und einem Katalysator hydriert und in eine Verbindung der Formel I umsetzt,
b) die erhaltene Verbindung der Formel I mit einem *tert.*-Butyldicarbonat und einem Acylierungskatalysator wie Dimethylaminopyridin (DMAP), in eine Verbindung der Formel IV umsetzt, 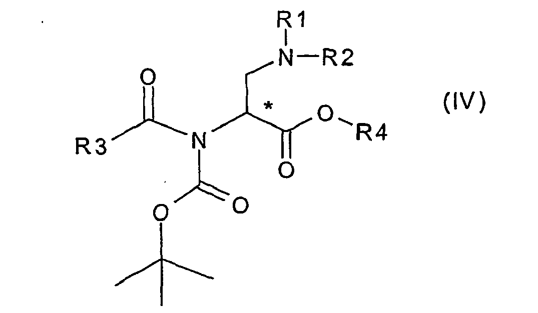
worin R1, R2, R3 und R4 die gleiche Bedeutung wie in der neuen Verbindung der Formel II haben.

Die Verbindungen der Formeln I, II, III und IV eignen sich als Zwischenverbindungen zur Herstellung von lkB-Kinase Inhibitoren (WO 01/30774 A1).

Nachfolgend ist die Erfindung an Hand von Beispielen näher erläutert.
Endprodukte werden in der Regel durch ¹H-NMR (400 MHz, in DMSO-D6) bestimmt, angegeben sind jeweils der Hauptpeak oder die beiden Hauptpeaks. Temperaturangaben in Grad Celsius, RT bedeutet Raumtemperatur (22 °C bis 26 °C). Verwendete Abkürzungen sind entweder erläutert oder entsprechen den üblichen Konventionen.

### Beispiel 1

### Herstellung von 2-Benzoylamino-3-diphenylamino-acrylsäuremethylester

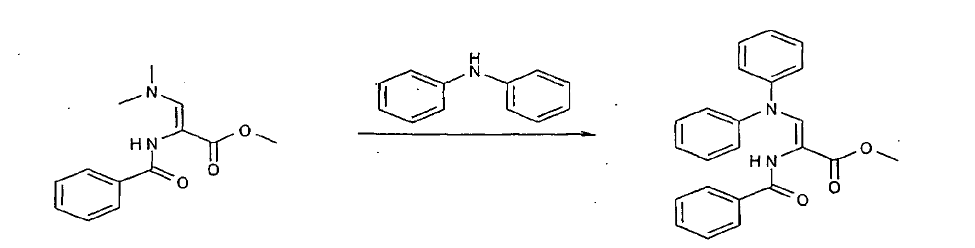

66 g (266 mmol) 2-Benzoylamino-3-dimethylamino-acrylsäuremethylester und 50 g (295 mmol) Diphenylamin wurden in 1300 ml Isopropanol bei 40 °C gelöst. Die Lösung wurde mit 60 ml (725 mmol) konzentrierter Salzsäure innerhalb von 5 Minuten (min) versetzt und 10 min nachgerührt. Es wurden 550ml Lösungsmittel unter verminderten Druck abgedampft, die Suspension auf 10 °C abgekühlt und vom auskristallisiertem Produkt abfiltriert.
- Ausbeute:: 83,5g (84 % der Theorie)
- 1H-NMR:: 3,62 (s, 3H), 6,95-7,10 (m, 6H), 7,20-7,30 (m, 8H), 7,32-7,40 (m, 1 H), 7,61 (s, 1H), 8,70 (s, 1 H)

### Beispiel 2

### Herstellung von 2-Benzyloxycarbonylamino-3-diphenylamino-acrylsäuremethylester

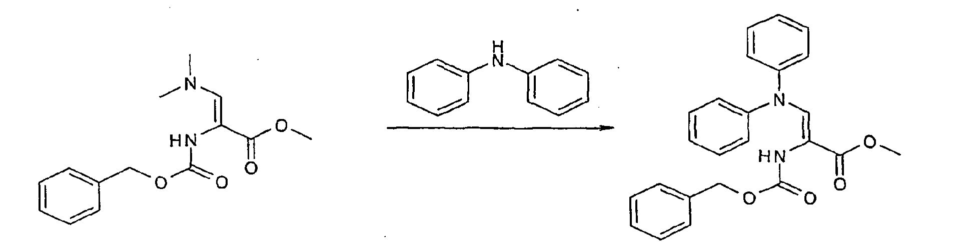

36 g (129 mmol) 2-Benzyloxycarbonylamino-3-dimethylaminoacrylsäuremethylester und 24,12 g (142 mmol) Diphenylamin wurden in 63 0ml Isopropanol bei 40 °C gelöst. Anschließend wurde die Lösung mit 17,4 ml konzentrierter Salzsäure innerhalb von 5 min versetzt und bei 40 °C 30 min nachgerührt. Die Reaktionslösung wurde auf 300 ml eingeengt und langsam mit 300 ml Wasser versetzt. Das auskristallisierte Produkt wurde abgesaugt und unter verminderten Druck bei 40 °C getrocknet.
- Ausbeute:: 30,5g (59 % der Theorie)
- 1H-NMR:: 3,62 (s, 3H), 4,68 (s, 2H), 6,95-7,10 (m, 6H), 7,20-7,50 (m, 9H), 7,61 (s, 1H)

### Beispiel 3

### Herstellung von racemischen 2-Benzoylamino-3-diphenylaminopropionsäuremethylester

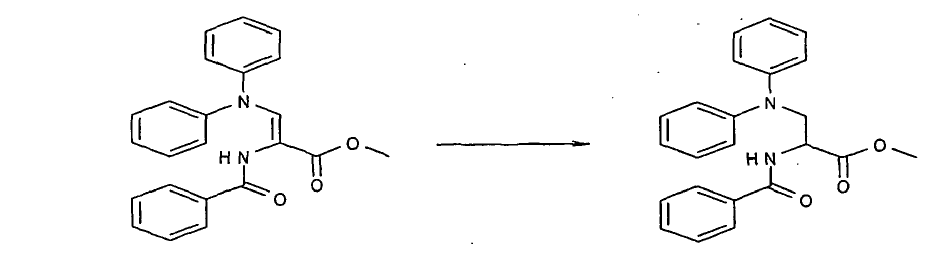

Ein Autoklav wurde unter Sauerstoffausschluss mit 1 g (2,68 mmol) 2-Benzoylamino-3-diphenylamino-acrylsäuremethylester und 40 mg (0,042 mmol) Tris(triphenylphosphin)-Rhodium(I)chlorid versehen. Nach Spülen mit Argon wurden 40 ml sauerstofffreies Methanol zugegeben. Der Autoklav wurde gasdicht verschlossen und die Lösung bei RT für 20 Stunden (h) hydriert. Der Autoklav wurde entspannt und mit Stickstoff gespült. Das Lösungsmittel wurde unter verminderten Druck abgedampft und der Rückstand über ein mit Kieslegel 60 gefüllte Säule chromatographiert (Eluent: Ethylaceta/Heptan 1:1). Nach Abdampfen der Lösungsmittel unter verminderten Druck verblieb ein weißer Feststoff der zur Ausarbeitung einer Methode und als Systemtest für die Bestimmung der Enantiomerenreinheit durch HPLC an chiraler Phase benutzt wurde.
- HPLC-Säule:: Chiralpak OD 4x250
- Eluent:: Hexan/EtOH/MeOH 45:2:1 + 0.1 % Diethylamin
- Temperatur:: 30°C
- Retentionszeit Edukt:: 13,2 Minuten
- Retentionszeit *S*-Isomer:: 11,8 Minuten
- Retentionszeit *R*-Isomer:: 14,2 Minuten

- Ausbeute:: 0,5 g (50% der Theorie)
- Ausbeute:: x 3,62 (s, 3H), 4,15-4,35 (m, 2H), 4,75-4,90 (m, 1H), 6,90-7,05 (m, 6H), 7,20-7,30 (m, 4H), 7,40-7,48 (m, 2H), 7,50-7,60 (m, 1H), 7,70 - 7,78 (d, 2H), 8,85 (d, 1H)

### Beispiel 4

### Herstellung von (S)-2-Benzoylamino-3-diphenylamino-propionsäuremethylester

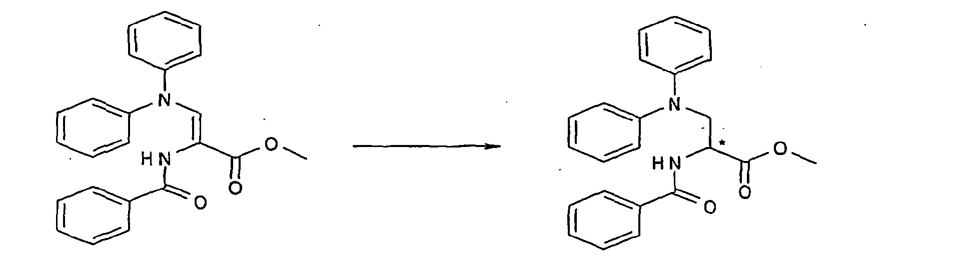

Eine Ampulle wurde unter Sauerstoff- und Feuchtigkeitsausschlussausschluss mit 100 mg 2-Benzoylamino-3-diphenylamino-acrylsäuremethylester (0,255 mmol) und 1,9 mg (0,0026 mmol, 0,01 Äquivalente) [(S,S)-Et-FerroTANE-Rh]BF₄ versehen. Nach Spülen mit Argon wurde 5 ml sauerstofffreies Methanol zugegeben. Die Ampulle wurde gasdicht verschlossen und in einem Autoklaven bei 20 bar Wasserstoffdruck für 24 h hydriert. Der Autoklav wurde entspannt und mit Stickstoff gespült. Der Umsatz der Hydrierung wurde durch HPLC bestimmt. Die Enantioselektivität wurde durch HPLC an chiraler Phase mit der in Beispiel 4 beschriebenen Methode bestimmt. Das [(*R*,*R*)-Et-FerroTANE-Rh]BF₄ Katalysator lieferte in gleicher Ausbeute und
- Enantiomerenreinheit die entsprechende R-Derivat.: ee: 87%

### Beispiel 5

### Herstellung von (S) und (R)-2-Benzoylamino-3-diphenylaminopropionsäuremethylester

Analog zu Beispiel 4 wurde 2-Benzoylamino-3-diphenylamino-acrylsäuremethylester mit verschiedenen Katalysatoren und Lösungsmittel hydriert. Bei den nicht vorgefertigten Katalysatoren, wurde der aktive Katalysator aus dem optisch aktiven Phosphin-Ligand und aquimolaren Mengen [Rh(cod)Cl]₂ als Rhodium(I)salz in situ hergestellt. Die Ergebnisse sind in der folgenden Tabelle 1 zusammengestellt.

**Tabelle 1:**

| VKS | Katalysator/Ligand | Rhodium-Salz | Druck [bar] | Lösungsmittel | Umsatz [%] | ee [%] |
|---|---|---|---|---|---|---|
| 1:100 | [(R,R)-Et-FerroTANE- Rh]BF₄ [268220-96-8] | nein | 20 | Toluol | 97 | 90 |
| 1:100 | [(R,R)-Et-FerroTANE- Rh]BF₄ [268220-96-8] | nein | 20 | Methanol | 95 | R 87 |
| 1:100 | [(R,R)-Et-FerroTANE- Rh]BF₄ [268220-96-8] | nein | 20 | Dichlormethan | 97 | 85 |
| 1:100 | [(S,S)-Et-FerroTANE- Rh]BF₄ [268220-96-8] | nein | 20 | Methanol | 94 | S 86 |
| 1:100 | [(R,R)-Me-DUPHOS- Rh]CF₃SO₃ [136705-77-6] | nein | 20 | Toluol | kU | nb |
| 1:100 | [(R,R)-Me-DUPHOS- Rh]CF₃SO₃ [136705-77-6] | nein | 20 | Methanol | 71 | 90 |
| 1:100 | [(R,R)-Me-DUPHOS- Rh]CF₃SO₃ [136705-77-6] | nein | 20 | Dichlormethan | 65 | 86 |
| 1:100 | (R)-(S)-JOSIPHOS [155806-35-2] | ja | 20 | Toluol | 68 | 36 |
| 1:100 | (R)-(S)-JOSIPHOS [155806-35-2] | ja | 20 | Methanol | 91 | 31 |
| 1:100 | (R)-(S)-JOSIPHOS [155806-35-2] | ja | 20 | Dichlormethan | 82 | 11 |
| 1:100 | L-BPPM-E [61478-28-2] | ja | 20 | Toluol | 21 | 68 |
| 1:100 | L-BPPM-E [61478-28-2] | ja | 20 | Methanol | 80 | 35 |
| 1:100 | L-BPPM-E [61478-28-2] | ja | 20 | Dichlormethan | 5 | 45 |
| 1:100 | (S)-BINAPHANE [544461- 38-3] | ja | 20 | Toluol | 37 | 6 |
| 1:100 | (S)-BINAPHANE [544461- 38-3] | ja | 20 | Methanol | 31 | 48 |
| 1:100 | (S)-BINAPHANE [544461- 38-3] | ja | 20 | Dichlormethan | 19 | 46 |
| 1:100 | (R)-(-)-tert.-Ferro [155830-69-6] | ja | 20 | Methanol | 59 | 99 |
| 1:100 | (R)-(-)-cyclohexyl.-Ferro [167416-28-6] | ja | 20 | Methanol | 11 | 99 |
| 1:100 | (R,R)-BDPP [96183-46-9] | ja | 20 | Methanol | kÜ | nb |
| 1:100 | (S,S)-CHIRAPHOS [64896-28-2] | ja | 20 | Methanol | kU | nb |
| 1:100 | (R,R)-DIOP [32305-98-9] | ja | 20 | Methanol | 5 | 95 |
| 1:100 | (R)-PROPHOS [67884-32-6] | ja | 20 | Methanol | kU | nb |
| 1:100 | (S,S)-NORPHOS [71042-55-2] | ja | 20 | Methanol | kU | nb |
| 1:100 | (R,R)-iPr-DUPHOS [136705-65-2] | ja | 20 | Methanol | 30 | 99 |
| 1:100 | [(R,R)-Et-BPE [136705-62-9] | ja | 20 | Methanol | 62 | 99 |
| 1:100 | [(R,R)-Me-BPE-Rh]CF₃SO₃ [213343-69-2] | nein | 20 | Methanol | 55 | 96 |
| 1:100 | (R)-Me-BOPHOZ [406680-93-1] | ja | 20 | Methanol | kU | nb |
| 1:100 | MonoPhos [157488-65-8] | ja | 20 | Methanol | kU | nb |
| 1:100 | MonoPhos [157488-65-8] | ja | 20 | Dichlormethan | kU | nb |
| 1:100 | MonoPhos [490023-37-5] | ja | 20 | Methanol | kU | nb |
| 1:100 | MonoPhos [490023-37-5] | ja | 20 | Dichlormethan | kU | nb |
| 1:100 | MonoPhos [380230-02-4] | ja | 20 | Methanol | kU | nb |
| 1:100 | MonoPhos [380230-02-4] | ja | 20 | Dichlormethan | kU | nb |

| | | | | | | |
|---|---|---|---|---|---|---|
| VKS = molares Verhältnis Katalysator zu Substrat KU = kein Umsatz Nb = nicht bestimmt R oder S in der Spalte "ee [%]" bedeutet das jeweilige R oder S Enantiomer | | | | | | |

### Beispiel 6

Ein Autoklav wurde unter Sauerstoffausschluss mit der in Tabelle 1 angegebenen Mengen von 2-Benzoylamino-3-diphenylamino-acrylsäuremethylester und [(R,R)-Me-DUPHOS-Rh]CF₃SO₃ versehen. Nach Spülen mit Argon wurde die unten angegebene Menge sauerstofffreies Methanol zugegeben. Der Autoklav wurde gasdicht verschlossen und die Lösung bei RT für 20 h bei 30 bar Wasserstoffdruck hydriert. Der Autoklav wurde entspannt und mit Stickstoff gespült. Der Umsatz der Hydrierung wurde durch HPLC bestimmt. Die Enantioselektivität wurde durch HPLC an chiraler Phase mit der in Beispiel 4 beschriebenen Methode bestimmt. Das Ergebnis wird in Tabelle 2 dargestellt.

**Tabelle 2:**

| Verhältnis Katalysator/Substrat | Katalysator | Edukt [g] | Umsatz [%] | ee [%] |
|---|---|---|---|---|
| 1 : 1000 | [(R,R)-Me-DUPHOS-Rh]CF₃SO₃ | 26 | 25 | 87 |

### Beispiel 7

Ein Autoklav wurde unter Sauerstoffausschluss mit der in Tabelle 2 angegebenen Mengen von 2-Benzoylamino-3-diphenylamino-acrylsäuremethylester und [(S,S)-Et-FerroTANE-Rh]BF₄ versehen. Nach Spülen mit Argon wurde die unten angegebene Menge sauerstofffreies Methanol zugegeben. Der Autoklav wurde gasdicht verschlossen und die Lösung bei RT für 20 h bei 30 bar Wasserstoffdruck hydriert. Der Autoklav wurde entspannt und mit Stickstoff gespült. Die Lösung wurde filtriert, bei 40 °C mit der gleichen Menge Wasser versetzt und 2 h bei RT nachgerührt. Das auskristallisierte Produkt wird abgesaugt und unter verminderten Druck bei 45 °C bis zur Gewichtskonstanz getrocknet. Der Umsatz der Hydrierung wurde durch HPLC bestimmt. Die Enantioselektivität wurde durch HPLC an chiraler Phase mit der in Beispiel 4 beschriebenen Methode bestimmt. Das Ergebnis wird in Tabelle 3 dargestellt.

**Tabelle 3:**

| Verhältnis Katalysator/Substrat | Katalysator | Edukt [g] | Umsatz [%] | ee [%] | Ausbeute [%] |
|---|---|---|---|---|---|
| 1:1000 | [(S,S)-Et-FerroTANE-Rh]BF₄ [268220-96-8] | 26 | 98 | 85 | 89 |
| 1:2500 | [(S,S)-Et-FerroTANE-Rh]BF₄ [268220-96-8] | 26 | 99 | 84 | 88 |
| 1:5000 | [(S,S)-Et-FerroTANE-Rh]BF₄ [268220-96-8] | 26 | 98 | 86 | 90 |
| 1 : 10000 | [(S,S)-Et-FerroTANE-Rh]BF₄ [268220-96-8] | 26 | 73 | 85 | n.i. |
| 1 : 5000 | [(S,S)-Et-FerroTANE-Rh]BF₄ [268220-96-8] | 260 | 98 | 85 | 89 |

| | | | | | |
|---|---|---|---|---|---|
| n. i. = nicht isoliert | | | | | |

### Beispiel 8

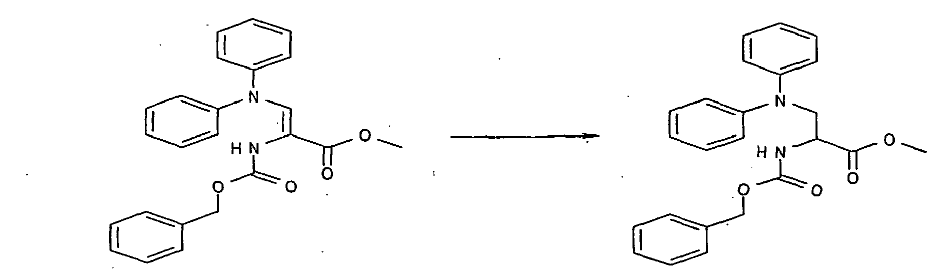

Ein Autoklav wurde unter Sauerstoffausschluss mit 1 g (2,68 mmol) 2-Benzyloxycarbonylamino-3-diphenylamino-acrylsäuremethylester und 40 mg (0,042 mmol) Tris(triphenylphosphin)-Rhodium(I)chlorid versehen. Nach Spülen mit Argon wurden 40 ml sauerstofffreies Methanol zugegeben. Der Autoklav wurde gasdicht verschlossen und die Lösung bei RT für 20 h hydriert. Der Autoklav wurde entspannt und mit Stickstoff gespült. Das Lösungsmittel wurde unter verminderten Druck abgedampft und der Rückstand über ein mit Kieslegel 60 gefüllte Säule gereinigt (Eluent: Ethylacetat/Heptan 1:1). Nach Abdampfen der Lösungsmittel unter verminderten Druck verblieb ein weißer Feststoff der zur Ausarbeitung einer Methode und als Systemtest für die Bestimmung der Enantiomerenreinheit durch HPLC an chiraler Phase benutzt wurde.
- HPLC-Säule:: Chiralpak OD 4x250
- Eluent:: Hexan/EtOH/MeOH 50:2:1 + 0.1 % Diethylamin
- Temperatur:: 30°C
- Retentionszeit Edukt:: 19,2 Minuten
- Retentionszeit S-Isomer:: 14,6 Minuten
- Retentionszeit R-Isomer:: 16,0 Minuten

- Ausbeute:: 0,2 g (20 % der Theorie)
- 1H-NMR:: 3,60 (s, 3H), 3,95-4,15 (m, 2H), 4,35-4,45 (m, 1H), 4,92-5,05 (m, 2H), 6,90-7,00 (m, 6H), 7,15-7,40 (m, 9H), 7,85-7,90 (d, 1H)

### Beispiel 9

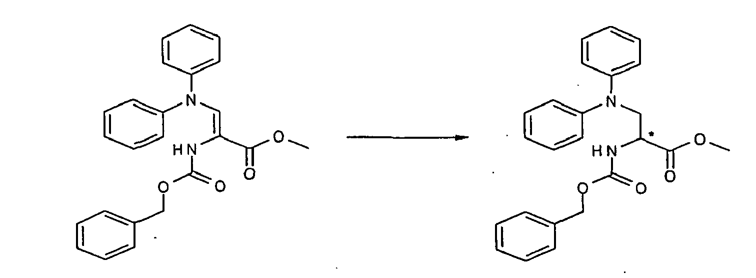

Analog zu Beispiel 5 wurde 2-Benzyloxycarbonylamino-3-diphenylamino-acrylsäuremethylester mit verschiedenen Katalysatoren und Lösungsmittel hydriert. Bei den nicht vorgefertigten Katalysatoren, wurde der aktive Katalysator aus dem optisch aktiven Phosphin-Ligand und aquimolaren Mengen [Rh(cod)Cl]₂ als Rhodium(I)salz in situ hergestellt. Die Ergebnisse sind in der folgenden Tabelle 4 zusammengestellt.

**Tabelle 4:**

| VKS | Katalysator/Ligand | Rhodium-Salz | Druck [bar] | Lösungsmittel | Umsatz [%] | ee [%] |
|---|---|---|---|---|---|---|
| 1:100 | [(R,R)-Et-FerroTANE-Rh]BF₄ [268220-96-8] | nein | 20 | Toluol | 15 | 43 |
| 1:100 | [(R,R)-Et-FerroTANE-Rh]BF₄ [268220-96-8] | nein | 20 | Methanol | 2 | nb |
| 1:100 | [(R,R)-Et-FerroTANE-Rh]BF₄ [268220-96-8] | nein | 20 | Dichlormethan | 4 | nb |
| 1:100 | [(R,R)-Me-DUPHOS-Rh]CF₃SO₃ [136705-77-6] | nein | 20 | Toluol | 15 | nb |
| 1:100 | [(R,R)-Me-DUPHOS-Rh]CF₃SO₃ [136705-77-6] | nein | 20 | Methanol | 25 | 61 |
| 1:100 | [(R,R)-Me-DUPHOS-Rh]CF₃SO₃ [136705-77-6] | nein | 20 | Dichlormethan | 29 | 46 |
| 1:100 | (R)-(S)-JOSIPHOS [155806-35-2] | ja | 20 | Toluol | 13. | nb |
| 1:100 | (R)-(S)-JOSIPHOS [155806-35-2] | ja | 20 | Methanol | 35 | 28 |
| 1:100 | (R)-(S)-JOSIPHOS [155806-35-2] | ja | 20 | Dichlormethan | 17 | 43 |
| 1:100 | L-BPPM-E [61478-28-2] | ja | 20 | Toluol | 5 | nb |
| 1:100 | L-BPPM-E [61478-28-2] | ja | 20 | Methanol | 7 | nb |
| 1:100 | L-BPPM-E [61478-28-2] | ja | 20 | Dichlormethan | 6 | nb |
| 1:100 | (S)-BINAPHANE [544461-38-3] | ja | 20 | Toluol | <5 | nb |
| 1:100 | (S)-BINAPHANE [544461-38-3] | ja | 20 | Methanol | <5 | nb |
| 1:100 | (S)-BINAPHANE [544461-38-3] | ja | 20 | Dichlormethan | <5 | nb |
| 1:100 | [(R,R)-Me-BPE-Rh]CF3SO3 | nein | 20 | Methanol | 36 | 73 |
| 1:100 | (R)-(-)-tert.-Ferro [155830-69-6] | ja | 20 | Methanol | 85 | 21 |
| 1:100 | (R)-(S)-JOSIPHOS | ja | 20 | Methanol | 99 | 24 |
| 1:100 | [(R,R)-Et-FerroTANE-Rh]BF4 | ja | 20 | Methanol | 52 | 60 |
| 1:100 | (R,R)-BDPP [96183-46-9] | ja | 20 | Methanol | kU | nb |
| 1:100 | (S,S)-CHIRAPHOS [64896-28-2] | ja | 20 | Methanol | kU | nb |
| 1:100 | (R,R)-DIOP [32305-98-9] | ja | 20 | Methanol | kU | nb |
| 1:100 | (R)-PROPHOS [67884-32-6] | ja | 20 | Methanol | kU | nb |
| 1:100 | (S,S)-NORPHOS [71042-55-2] | ja | 20 | Methanol | kU | nb |
| 1:100 | [(R,R)-Et-BPE-Rh]BF4 | ja | 20 | Methanol | 6 | 23 |
| 1:100 | (R)-Me-BOPHOZ (Eastman) | nein | 20 | Methanol | 25 | 16 |

| | | | | | | |
|---|---|---|---|---|---|---|
| VKS = Verhältnis Katalysator zu Substrat; KU = kein Umsatz Nb = nicht bestimmt; < = kleiner als | | | | | | |

### Beispiel 10 Herstellung von (S) 2-(Benzoyl-tert-butoxycarbonyl-amino)-3-diphenylamino-propionsäure-methylester

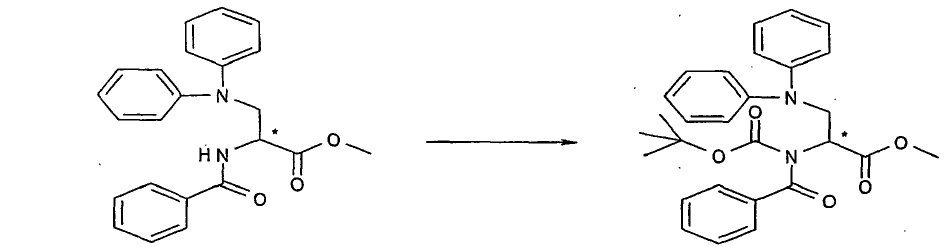

18,7g (*S*) 2-(Benzoyl-tert-butoxycarbonyl-amino)-3-diphenylamino-propionsäuremethylester, ee=85%, 20,6 Di-tert.-Butyldicarbonat und 1,2g N,N-Dimethylaminopyridin wurden in 90 ml Acetonitril gelöst und 3 Stunden bei 40°C gerührt. Das Acetonitril wurde unter verminderten Druck abgedampft und der verbleibende Rückstand in 300ml Düsopropylether aufgenommen und heiß filtriert. Das Produkt kristallisierte als farbloser Feststoff über Nacht aus.
- Ausbeute:: 23,7 g (88% der Theorie)
- 1H-NMR:: 1,38 (s, 9H), 3,70 (s, 3H), 4,35-4,58 (m, 2H), 5,45-5,52 (m, 1H), 6,93-7,05 (m, 6H), 7,13-7,18 (m, 2H), 7,22-7,30 (m, 4H), 7,32 - 7,30 (m, 2H), 7,45-7,52 (m, 1H)

### Beispiel 11 Herstellung von (S) 2-( tert-butoxycarbonyl-amino)-3-diphenylaminopropionsäure-methylester

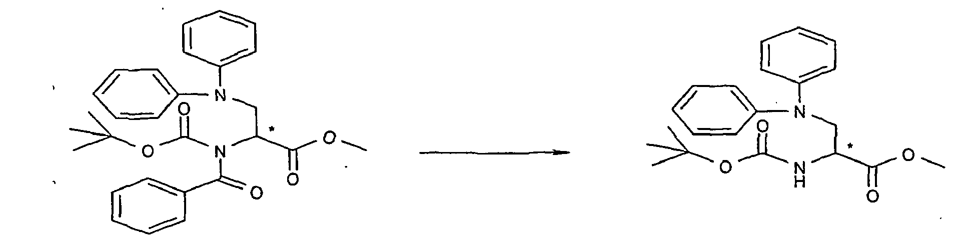

1,3g (S) 2-(Benzoyl-tert-butoxycarbonyl-amino)-3-diphenylamino-propionsäuremethylester wurden in 13ml Methanol gelöst und mit 2,74ml einer 1 M Lösung von Magnesiummethylat in Methanol versetzt. Die Lösung wurde über Nacht bei RT gerührt und unter verminderten Druck eingeengt. Der Rückstand wurde in Ethylacetat aufgenommen und mit Wasser gewaschen. Die Ethylacetatphase wurde über Natriumsulfat getrocknet, filtiert und unter verminderten Druck eingedampft. Der Rückstand wurde aus wenig Diisoprpylether/Heptan kristallisiert.
- Ausbeute:: 0,95 g (90% der Theorie)
- 1H-NMR:: 1,38 (s, 9H), 3,55 (s, 3H), 4,10-4,25 (m, 2H), 4,50-4,62 (m, 1H), 5,10-5,25 (m, 1H), 6,90-7,05 (m, 6H), 7,20-7,30 (m, 4H)

### Beispiel 12 Herstellung von (S) 2 -Amino-3-diphenylamino-propionsäure-methylester p-Toluolsulfonat

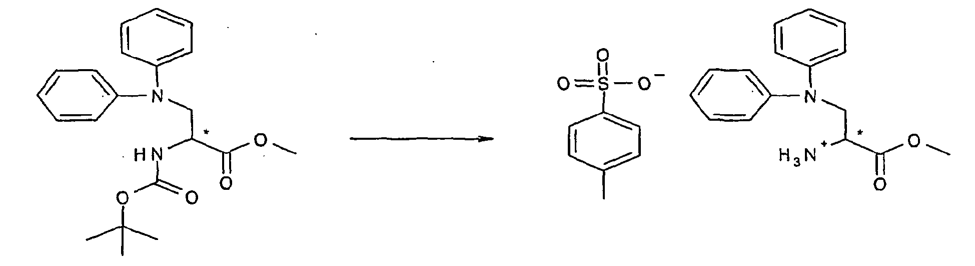

18,5g (S) 2-( tert-butoxycarbonyl-amino)-3-diphenylamino-propionsäure-methylester (ee=85%) wurden in 100ml Dichlormethan gelöst und mit und mit 50ml Trifluoressigsäure (TFA) versetzt. Die Lösung wurde für 30 Minuten unter Rückfluss erhitzt und anschließend unter verminderten Druck auf 100ml Volumen eingeengt. Die Lösung wurde mit Wasser gewaschen und mit 9g p-Toluolsulfonsäure versetzt. Es wurden 125ml n-Butanol zugeben und das verbliebene Dichlormethan abgedampft. Zur Kristallisation des p-Toluolsulfonsäuresalzes wurde die Lösung auf RT abgekühlt und über Nacht nachgerührt. Der Feststoff wurde abgesaugt und unter verminderten Druck bis zur Gewichtskonstanz getrocknet.
- Ausbeute:: 16,6 g (81 % der Theorie, bezogen auf gewünschtes Isomer)
- 1H-NMR:: 2,38 (s, 3H), 3,30 (s, 3H), 4,10-4,35 (m, 3H), 5,45-5,52 (m, 1H), 6,73-6,95 (m, 6H), 7,01-7,05 (m, 2H), 7,10-7,18 (m, 4H), 7,58 - 7,62 (m, 2H), 8,30-8,55 (s, breit, 3H, NH)
- ee:: 99%

### Beispiel 13

### Herstellung von 2-Benzoylamino-3-phenylamino-acrylsäuremethylester

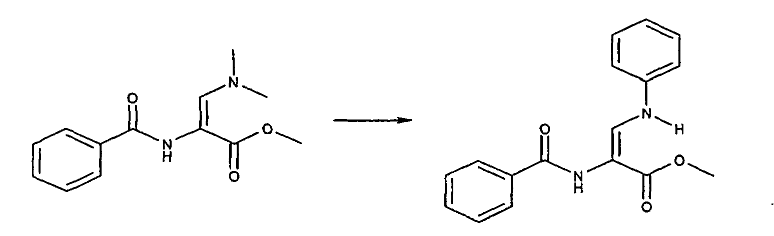

10 g (39,5 mmol) 2-Benzoylamino-3-dimethylamino-acrylsäuremethylester und 11,1 g (118 mmol) Anilin wurden in 200 ml Isopropanol bei 40 °C gelöst. Die Lösung wurde mit 3,6 ml (43,5 mmol) konzentrierter Salzsäure innerhalb von 5 Minuten (min) versetzt und 10 min nachgerührt. Es wurden 200ml entionisiertes Wasser zugegeben, die Suspension auf 10 °C abgekühlt und vom auskristallisiertem Produkt abfiltriert.
- Ausbeute:: 11,5g (92 % der Theorie)
- 1H-NMR:: 3,62 (s, 3H); 6,90-7,00 (m, 1H); 7,19 (d, 2H); 7,25-7,30 (m, 2H); 7,48-7,61 (m, 3H); 7,93 (d, 1H); 8,02 (d, 2H); 8,90 (d, 1H); 9,15 (s, 1H)

### Beispiel 14

### Herstellung von 2-Benzoylamino-3-(4-fluorphenylamino)-acrylsäuremethylester

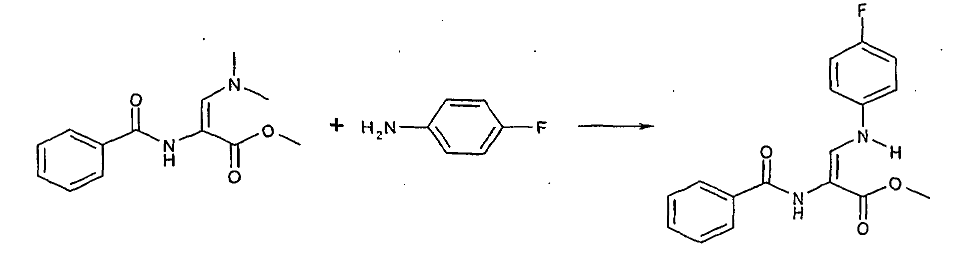

10 g (39,5 mmol) 2-Benzoylamino-3-dimethylamino-acrylsäuremethylester und 11,4 ml (118 mmol) 4-Fluoranilin wurden in 200 ml Isopropanol bei 40 °C gelöst. Die Lösung wurde mit 3,6 ml (43,5 mmol) konzentrierter Salzsäure innerhalb von 5 Minuten (min) versetzt und 30 min nachgerührt. Die Suspension wurde auf 10 °C abgekühlt und vom auskristatlisiertem Produkt abfiltriert.
- Ausbeute:: 12,4g (94 % der Theorie)
- 1H-NMR:: 3,62 (s, 3H); 7,05-7,24 (m, 4H); 7,48-7,52 (m, 3H); 7,88 (d, 1H); 8,00-8,04 (m, 2H); 8,90 (d, 1H); 9,15 (s, 1H)

### Beispiel 15

### Herstellung von 2-Benzoylamino-3-(pyridin-2-ylamino)-acrylsäuremethylester

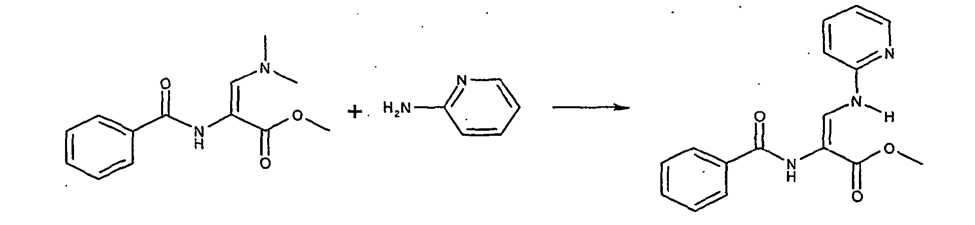

10 g (39,5 mmol) 2-Benzoylamino-3-dimethylamino-acrylsäuremethylester und 11,3 g (118 mmol) 2-Aminopyridin wurden in 200 ml Isopropanol bei 40 °C gelöst. Die Lösung wurde mit 3,96 ml (48 mmol) konzentrierter Salzsäure innerhalb von 5 Minuten (min) versetzt und 30 min nachgerührt. Die Suspension wurde auf 10 °C abgekühlt und vom auskristallisiertem Produkt abfiltriert.
- Ausbeute:: 7,3g (60 % der Theorie)
- 1H-NMR:: 3,62 (s, 3H); 6,92-6,97 (m, 1H); 7,02 (d, 1H); 7,45-7,70 (m, 4H); 8,02 (d, 2H); 8,22-8,24 (m, 1H); 8,60 (d, 1H); 9,22 (s, 1H); 9,45 (d, 1H)

### Beispiel 16

### Herstellung von 2-Benzoylamino-3-(thiazol-2-ylamino)-acrylsäuremethylester

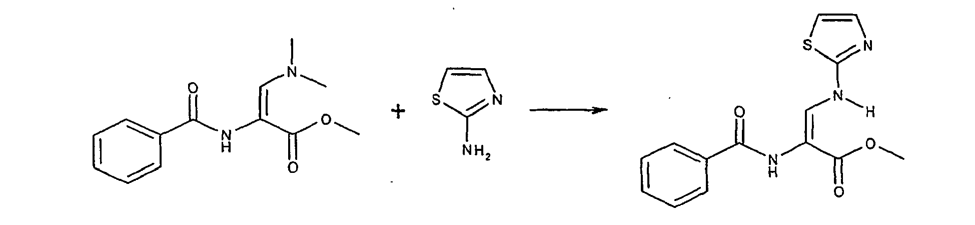

10 g (39,5 mmol) 2-Benzoylamino-3-dimethylamino-acrylsäuremethylester und 11,3 g (118 mmol) 2-Aminothiazol wurden in 200 ml Isopropanol bei 40 °C gelöst. Die Lösung wurde mit 3,96 ml (48 mmol) konzentrierter Salzsäure innerhalb von 5 Minuten (min) versetzt und 60 min nachgerührt. Es wurden 75ml entionisiertes Wasser zugegeben, die Suspension über Nacht abgekühlt und vom auskristallisiertem Produkt abfiltriert.
- Ausbeute:: 8,6g (70 % der Theorie)
- 1H-NMR:: 3,62 (s, 3H); 7,08 (d, 1H); 7,32 (d, 1H); 7,45-7,60 (m, 3H); 8,02 (d, 2H); 8,22 (d, 1H); 9,28 (s, 1H); 10,45 (d, 1H)

## Patentansprüche

1. Verbindungen der Formel II, 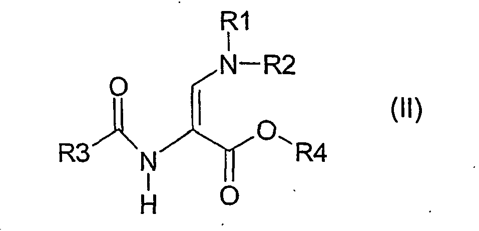 wobei R1 und R2 gleich oder verschieden sind und unabhängig voneinander für
1) -(C₆-C₁₄)-Aryl, wobei Aryl unsubstituiert oder unabhängig voneinander ein-, zwei- oder dreifach durch R11 substituiert ist,
wobei R11 für
a) F, Cl, J oder Br,
b) -(C₁-C₄)-Alkyl,
c) -CN,
d) -CF₃,
e) -OR⁵, worin R⁵ für Wasserstoffatom oder -(C₁-C₄)-Alkyl steht,
f) -N(R⁵)-R⁶, worin R⁵ und R⁶ unabhängig voneinander für Wasserstoffatom oder -(C₁-C₄)-Alkyl stehen,
g) -C(O)-R⁵, worin R⁵ für Wasserstoffatom oder -(C₁-C₄)-Alkyl steht, oder
h) -S(O)ₓ-R⁵, worin x die ganze Zahl Null, 1 oder 2 bedeutet und R⁵ Wasserstoffatom oder -(C₁-C₄)-Alkyl bedeutet, steht, oder
2) 4- bis 15-gliedriger Het-Ring, stehen, wobei der Het-Ring unsubstituiert ist oder ein-, zwei- oder dreifach unabhängig voneinander substituiert ist durch
-(C₁-C₅)-Alkyl, -(C₁-C₅)-Alkoxy, Halogen, Nitro, Amino, Trifluormethyl, Hydroxyl, Hydroxy-(C₁-C₄)-Alkyl, Methylendioxy, Ethylendioxy, Formyl, Acetyl, Cyano, Hydroxycarbonyl, Aminocarbonyl oder -(C₁-C₄)-Alkoxycarbonil
R3 für
1) -(C₆-C₁₄)-Aryl, worin Aryl unsubstituiert oder unabhängig voneinander
ein-, zwei- oder dreifach durch -NO₂, -O-(C₁-C₄)-Alkyl, F, Cl oder Brom substituiert ist,
2) -O-C(CH₃)₃, oder
3) -O-CH(R7)-Aryl, worin Aryl unsubstituiert oder unabhängig voneinander
ein-, zwei- oder dreifach durch -NO₂, -O-CH₃, F, Cl oder Brom substituiert ist,
worin R7 Wasserstoffatom oder -(C₁-C₄)-Alkyl bedeutet,
R4 für
1) Wasserstoffatom,
2) -(C₁-C₄)-Alkyl oder
3) -CH(R8)-Aryl steht,
worin R8 Wasserstoffatom oder -(C₁-C₄)Alkyl bedeutet.

2. Verbindung der Formel IV, 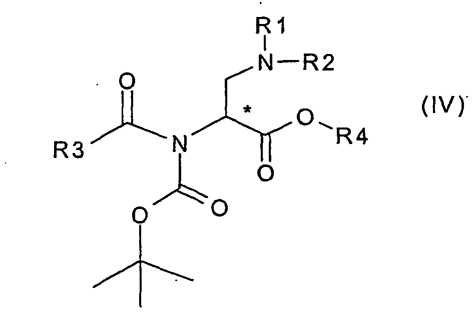 wobei R1, R2, R3 und R4 die gleiche Bedeutung haben wie in der Verbindung
der
Formel II gemäß Anspruch 1.

3. Verfahren zur Gewinnung der Verbindung der Formel I, 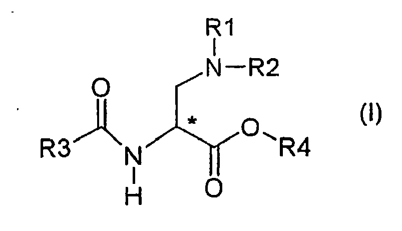 wobei R1 für
1) Wasserstoffatom,
2) -(C₁-C₄)-Alkyl,
3) -(C₆-C₁₄)-Aryl, wobei Aryl unsubstituiert oder unabhängig voneinander ein-, zwei- oder dreifach durch R11 substituiert ist, wobei R11 für
a) F, Cl, J oder Br,
b) -(C₁-C₄)-Alkyl,
c) -CN,
d) -CF₃,
e) -OR⁵, worin R⁵ für Wasserstoffatom oder -(C₁-C₄)-Alkyl steht,
f) -N(R⁵)-R⁶, worin R⁵ und R⁶ unabhängig voneinander für Wasserstoffatom oder -(C₁-C₄)-Alkyl stehen,
g) -C(O)-R⁵, worin R⁵ für Wasserstoffatom oder -(C₁-C₄)-Alkyl steht, oder
h) -S(O)ₓ-R⁵, worin x die ganze Zahl Null, 1 oder 2 bedeutet und R⁵ Wasserstoffatom oder -(C₁-C₄)-Alkyl bedeutet, steht,
4) -CH(R7)-Aryl, worin Aryl unsubstituiert oder unabhängig voneinander ein-zwei- oder dreifach durch -NO₂, -O-CH₃, F, Cl oder Brom substituiert ist, worin R7 Wasserstoffatom oder -(C₁-C₄) Alkyl bedeutet, oder
5) 4- bis 15-gliedriger Het-Ring, steht, wobei der Het-Ring unsubstituiert ist oder ein-, zwei- oder dreifach unabhängig voneinander substituiert ist durch -(C₁-C₅)-Alkyl, -(C₁-C₅)-Alkoxy, Halogen, Nitro, Amino, Trifluormethyl, Hydroxyl, Hydroxy-(C₁-C₄)-Alkyl, Methylendioxy, Ethylendioxy, Formyl, Acetyl, Cyano, Hydroxycarbonyl, Aminocarbonyl oder -(C₁ -C₄)-Alkoxycarbonyl,
R2 für
1) -(C₆-C₁₄)-Aryl, wobei Aryl unsubstituiert oder unabhängig voneinander ein-, zwei- oder dreifach durch R11 substituiert ist,
2) -CH(R7)-Aryl, worin Aryl unsubstituiert oder unabhängig voneinander ein- , zwei- oder dreifach durch -NO₂, -O-CH₃, F, Cl oder Brom substituiert ist, worin R7 Wasserstoffatom oder -(C₁-C₄)-Alkyl bedeutet, oder
3) 4- bis 15-gliedriger Het-Ring, steht, wobei der Het-Ring unsubstituiert ist oder ein-, zwei- oder dreifach unabhängig voneinander substituiert ist durch -(C₁-C₅)-Alkyl, -(C₁-C₅)-Alkoxy, Halogen, Nitro, Amino, Trifluormethyl, Hydroxyl, Hydroxy-(C₁-C₄)-Alkyl, Methylendioxy, Ethylendioxy, Formyl, Acetyl, Cyano, Hydroxycarbonyl, Aminocarbonyl oder -(C₁-C₄)-Alkoxycarbonyl,
R3 für
1) Wasserstoffatom,
2) -(C₁-C₄)-Alkyl,
3) -(C₆-C₁₄)-Aryl, worin Aryl unsubstituiert oder unabhängig voneinander ein-, zwei- oder dreifach durch -NO₂, -O-(C₁-C₄)-Alkyl, F, Cl oder Brom substituiert ist,
4) -O-C(CH₃)₃, oder
5) -O-CH(R7)-Aryl, worin Aryl unsubstituiert oder unabhängig voneinander ein-, zwei- oder dreifach durch -NO₂, -O-CH₃, F, Cl oder Brom substituiert ist,
worin R7 Wasserstoffatom oder -(C₁-C₄)-Alkyl bedeutet,
R4 für
1) Wasserstoffatom,
2) -(C₁-C₄)-Alkyl oder
3) -CH(R8)-Aryl steht,
worin R8 Wasserstoffatom oder -(C₁-C₄)-Alkyl bedeutet,
**dadurch gekennzeichnet, dass** man eine Verbindung der Formel II 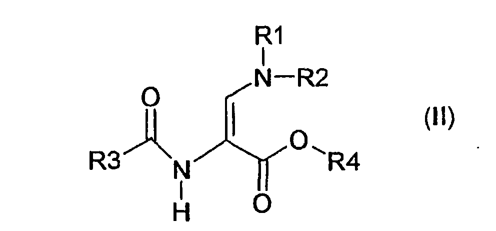 worin R1, R2, R3 und R4 die gleiche Bedeutung wie in der Formel I haben und die Verbindung der Formel II in der E als auch der Z Konfiguration an der Doppelbindung vorliegen kann,
in Anwesenheit von Wasserstoff und einem Katalysator hydriert.

4. Verfahren nach Anspruch 3, **dadurch gekennzeichnet, dass** man die Verbindung der Formel I gewinnt,
wobei R1 für Phenyl oder Wasserstoffatom steht,
R2 für Phenyl, Pyridyl oder Thiazolyl steht, worin Phenyl, Pyridyl oder Thiazolyl unsubstituiert oder durch Fluor oder Chlor substituiert sind, und
R3 für Phenyl oder -O-CH₂-Phenyl steht und
R4 für Methyl oder Ethyl steht.

5. Verfahren nach Anspruch 3 oder 4, **dadurch gekennzeichnet, dass** man als Katalysator Rhodium-, Ruthenium- oder Iridiumkomplexe oder Gemische davon einsetzt.

6. Verfahren nach einem oder mehreren der Ansprüche 3 bis 5, **dadurch gekennzeichnet, dass** das molare Verhältnis von Katalysator zur Verbindung der Formel II von 1 zu 100 bis 1 zu 10000 liegt.

7. Verfahren nach einem oder mehreren der Ansprüche 3 bis 6, **dadurch gekennzeichnet, dass** die Hydrierung bei einer Temperatur von 10 °C bis 200 °C und einem Wasserstoffdruck von 1 bar bis 200 bar durchgeführt wird.

8. Verfahren nach einem oder mehreren der Ansprüche 3 bis 7, **dadurch gekennzeichnet, dass** als Lösungsmittel Wasser, Alkohole wie Methanol, Ethanol, Propanol oder Isopropanol, aromatische Kohlenwasserstoffe wie Toluol, Ketone wie Aceton, halogenierte Kohlenwasserstoffe wie Dichlormethan, Carbonsäureester wie Ethylacetat und Ether wie Tetrahydrofuran oder Mischungen davon eingesetzt werden.

9. Verfahren zur Gewinnung der Verbindung der Formel III, 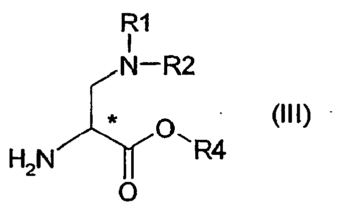 oder deren Salze, worin R1, R2 und R4 die gleiche Bedeutung wie in der
Formel I gemäß Anspruch 3 haben,
**dadurch gekennzeichnet, dass** man
a) die Verbindung der Formel II, 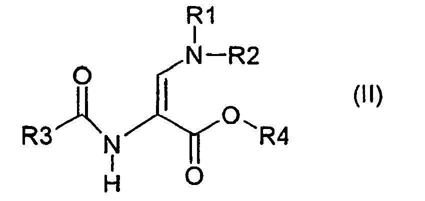 worin R1, R2, R3 und R4 die gleiche Bedeutung wie in der Formel I haben,
in Anwesenheit von Wasserstoff und einem Katalysator hydriert und in eine Verbindung der Formel I 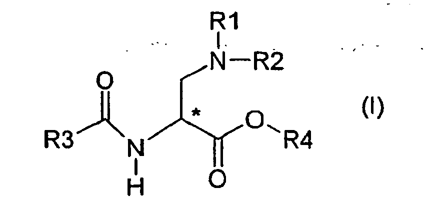 umsetzt, und
b) die erhaltene Verbindung der Formel I in eine Verbindung der Formel III überführt.

10. Verfahren zur Gewinnung der Verbindung der Formel III gemäß Anspruch 9,
**dadurch gekennzeichnet, dass** man
a) die Verbindung der Formel II,
worin R1, R2, R3 und R4 die gleiche Bedeutung wie in der Formel I haben,
in Anwesenheit von Wasserstoff und einem Katalysator hydriert und in eine Verbindung der Formel I umsetzt,
b) die erhaltene Verbindung der Formel I mit einem *tert*.-Butyldicarbonat und einem Acylierungskatalysator wie N,N-Dimethylaminopyridin in eine Verbindung der Formel IV umsetzt, 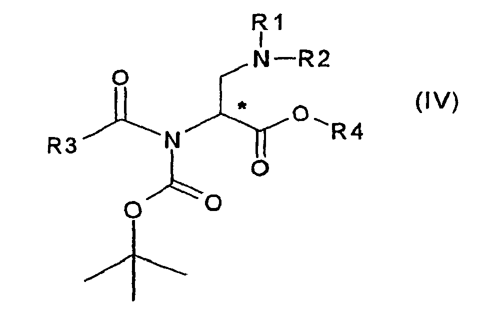 worin R1, R2, R3 und R4 die Bedeutung wie in Formel I haben, und
c) die erhaltene Verbindung der Formel IV anschließend in die Verbindung der Formel Ia 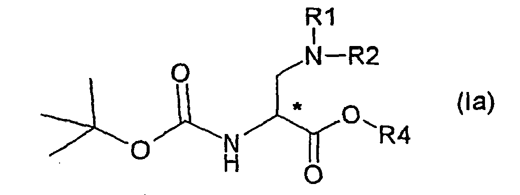 überführt und
d) die erhaltene Verbindung der Formel Ia in die Verbindung der Formel III oder deren Salze, überführt, worin R1, R2, und R4 die Bedeutung wie in Formel I haben.

11. Verfahren zur Gewinnung der Verbindung der Formel II gemäß Anspruch 1,
**dadurch gekennzeichnet, dass** man die Verbindung der Formel VI 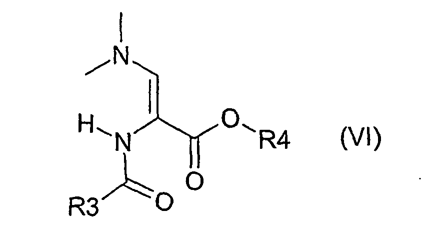 worin R3 und R4 die Bedeutung wie in Formel II gemäß Anspruch 1 Haben, mit einem Amin der Formel V,
R1-N(H)-R2 (V)
worin R1 und R2 die Bedeutung wie in Formel II gemäß Anspruch 1 haben, zu der Verbindung der Formel II umsetzt.

12. Verfahren zur Gewinnung der Verbindung der Formel IV gemäß Anspruch 2,
**dadurch gekennzeichnet, dass** man
a) die Verbindung der Formel II gemäß Anspruch 1,
worin R1, R2, R3 und R4 die gleiche Bedeutung wie in Anspruch 2 haben,
in Anwesenheit von Wasserstoff und einem Katalysator hydriert und in eine Verbindung der Formel I umsetzt, und
b) die erhaltene Verbindung der Formel I mit einem *tert*.-Butyldicarbonat und einem Acylierungskatalysator wie Dimethylaminopyridin in eine Verbindung der Formel IV umsetzt, 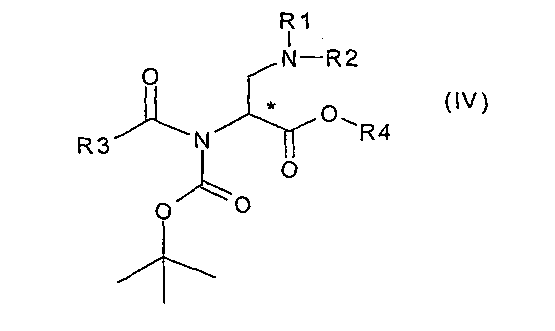
worin R1, R2, R3 und R4 die gleiche Bedeutung wie in Anspruch 2 haben.

## Claims

1. A compound of the formula II 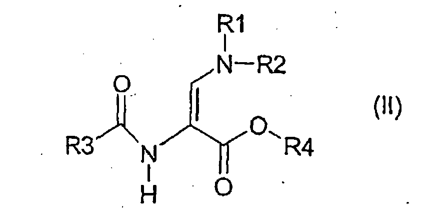 where R1 and R2 are the same or different and are each independently
1) -(C₆-C₁₄)-aryl where aryl is unsubstituted or mono-, di- or trisubstituted independently by R11,
where R11 is
a) F, CI, I or Br,
b) -(C₁-C₄)-alkyl,
c) -CN,
d) -CF₃,
e) -OR⁵ in which R⁵ is a hydrogen atom or -(C₁-C₄)-alkyl,
f) -N(R⁵)-R⁶ in which R⁵ and R⁶ are each independently a hydrogen atom or -(C₁-C₄)-alkyl,
g) -C(O)-R⁵ in which R⁵ is a hydrogen atom or -(C₁-C₄)-alkyl, or
h) -S(O)ₓ-R⁵ in which x is the integer zero, 1 or 2, and R⁵ is a hydrogen atom or -(C₁-C₄)-alkyl, or
2) a 4- to 15-membered Het ring where the Het ring is unsubstituted or mono-, di- or trisubstituted independently by -(C₁-C₅)-alkyl, -(C₁-C₅)-alkoxy, halogen, nitro, amino, trifluoromethyl, hydroxyl, hydroxy-(C₁-C₄)-alkyl, methylenedioxy, ethylenedioxy, formyl, acetyl, cyano, hydroxycarbonyl, amino-carbonyl or -(C₁-C₄)-alkoxycarbonyl,
R3 is
1) -(C₆-C₁₄)-aryl in which aryl is unsubstituted or mono-, di- or trisubstituted independently by -NO₂, -O-(C₁-C₄)-alkyl, F, Cl or bromine,
2) -O-C(CH₃)₃, or
3) -O-CH(R7)-aryl in which aryl is unsubstituted or mono-, di- or trisubstituted independently by -NO₂, -O-CH₃, F, Cl or bromine, in which R7 is a hydrogen atom or -(C₁-C₄)-alkyl,
R4 is
1) a hydrogen atom,
2) -(C₁-C₄)-alkyl or
3) -CH(R8)-aryl
in which R8 is a hydrogen atom or -(C₁-C₄)-alkyl.

2. A compound of the formula IV 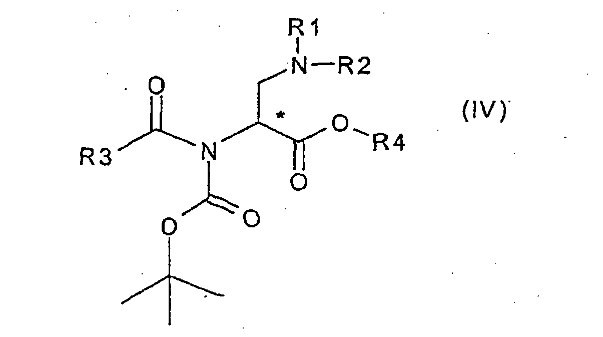 where R1, R2, R3 and R4 are each as defined as in the compound of the formula II as claimed in claim 1.

3. A process for obtaining the compound of the formula I 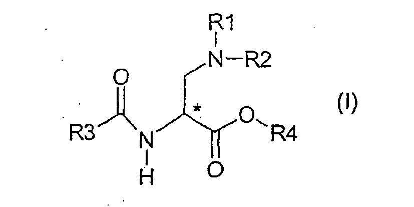 where R1 is
1) a hydrogen atom,
2) -(C₁-C₄)-alkyl,
3) -(C₆-C₁₄)-aryl where aryl is unsubstituted or mono-, di- or trisubstituted independently by R11, where R11 is
a) F, Cl, I or Br,
b) -(C₁-C₄)-alkyl,
c) -CN,
d) -CF₃,
e) -OR⁵ in which R⁵ is a hydrogen atom or -(C₁-C₄)-alkyl,
f) -N(R⁵)-R⁶ in which R⁵ and R⁶ are each independently a hydrogen atom or -(C₁-C₄)-alkyl,
g) -C(O)-R⁵ in which R⁵ is a hydrogen atom or -(C₁-C₄)-alkyl, or
h) -S(O)ₓ-R⁵ in which x is the integer zero, 1 or 2, and R⁵ is a hydrogen atom or -(C₁-C₄)-alkyl,
4) -CH(R7)-aryl in which aryl is unsubstituted or mono-, di- or trisubstituted independently
by -NO₂, -O-CH₃, F, Cl or bromine, where R7 is a hydrogen atom or -(C₁-C₄)-alkyl, or
5) a 4- to 15-membered Het ring where the Het ring is unsubstituted or mono-, di- or trisubstituted independently by -(C₁-C₅)-alkyl, -(C₁-C₅)-alkoxy, halogen, nitro, amino, trifluoromethyl, hydroxyl, hydroxy-(C₁-C₄)-alkyl, methylenedioxy, ethylenedioxy, formyl, acetyl, cyano, hydroxycarbonyl, aminocarbonyl or -(C₁-C₄)-alkoxycarbonyl,
R2 is
1) -(C₆-C₁₄)-aryl where aryl is unsubstituted or mono-, di-or trisubstituted independently by R11,
2) -CH(R7)-aryl in which aryl is unsubstituted or mono-, di- or trisubstituted independently by -NO₂ -O-CH₃, F, Cl or bromine, where R7 is a hydrogen atom or -(C₁-C₄)-alkyl, or
3) a 4- to 15-membered Het ring where the Het ring is unsubstituted or mono-, di- or trisubstituted independently by -(C₁-C₅)-alkyl, -(C₁-C₅)-alkoxy, halogen, nitro, amino, trifluoromethyl, hydroxyl, hydroxy-(C₁-C₄)-alkyl, methylenedioxy, ethylenedioxy, formyl, acetyl, cyano, hydroxycarbonyl, aminocarbonyl or -(C₁-C₄)-alkoxycarbonyl ,
R3 is
1) a hydrogen atom,
2) -(C₁-C₄)-alkyl,
3) -(C₆-C₁₄)-aryl in which aryl is unsubstituted or mono-, di- or trisubstituted independently by -NO₂ -O-(C₁-C₄)-alkyl, F, Cl or bromine,
4) -O-C(CH₃)₃, or
5) -O-CH(R7)-aryl in which aryl is unsubstituted or mono-, di- or trisubstituted independently by -NO₂, -O-CH₃, F, Cl or bromine, where R7 is a hydrogen atom or -(C₁-C₄)-alkyl,
R4 is
1) a hydrogen atom,
2) -(C₁-C₄)-alkyl or
3) -CH(R8)-aryl in which R8 is a hydrogen atom or -(C₁-C₄)-alkyl,
which comprises hydrogenating a compound of the formula II 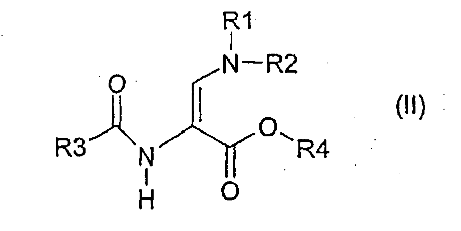 in which R1, R2, R3 and R4 are each as defined in the formula I and the compound of the formula II may be present in the E or in the Z configuration on the double bond,
in the presence of hydrogen and a catalyst.

4. The process as claimed in claim 3, wherein the compound of the formula I is obtained
where R1 is phenyl or a hydrogen atom,
R2 is phenyl, pyridyl or thiazolyl, in which phenyl, pyridyl or thiazolyl is unsubstituted or substituted by fluorine or chlorine, and
R3 is phenyl or -O-CH₂-phenyl and
R4 is methyl or ethyl.

5. The process as claimed in claim 3 or 4, wherein the catalyst used comprises rhodium complexes, ruthenium complexes or iridium complexes or mixtures thereof.

6. The process as claimed in one or more of claims 3 to 5, wherein the molar ratio of catalyst to the compound of the formula II is from 1:100 to 1:10 000.

7. The process as claimed in one or more of claims 3 to 6, wherein the hydrogenation is performed at a temperature of from 10°C to 200°C and a hydrogen pressure of from 1 bar to 200 bar.

8. The process as claimed in one or more of claims 3 to 7, wherein the solvent used comprises water, alcohols such as methanol, ethanol, propanol or isopropanol, aromatic hydrocarbons such as toluene, ketones such as acetone, halogenated hydrocarbons such as dichloromethane, carboxylic esters such as ethyl acetate, and ethers such as tetrahydrofuran, or mixtures thereof.

9. A process for obtaining the compound of the formula III 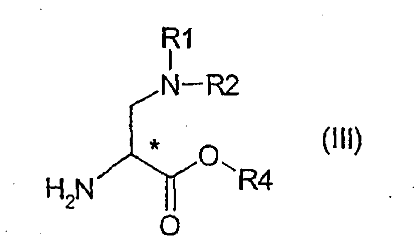 or salts thereof in which R1, R2 and R4 are each as defined in the formula I as claimed in claim 3,
which comprises
a) hydrogenating the compound of the formula II 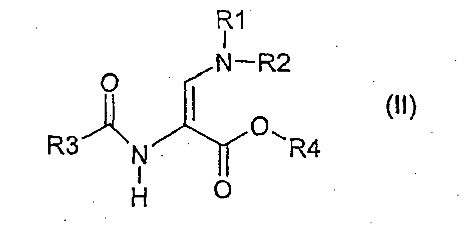 in which R1, R2, R3 and R4 are each as defined in the formula I,
in the presence of hydrogen and a catalyst, and converting it to a compound of the formula I 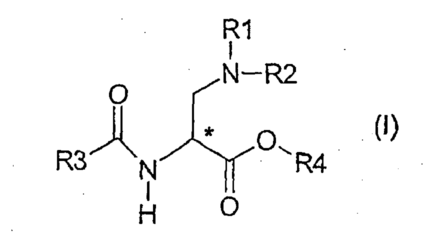 and
b) converting the resulting compound of the formula I to a compound of the formula III.

10. The process for obtaining the compound of the formula III as claimed in claim 9, which comprises
a) hydrogenating the compound of the formula II
in which R1, R2, R3 and R4 are each as defined in the formula I
in the presence of hydrogen and a catalyst and converting it to a compound of the formula I,
b) reacting the resulting compound of the formula I with a tert-butyl dicarbonate and an acylation catalyst such as N,N-dimethylaminopyridine to give a compound of the formula IV 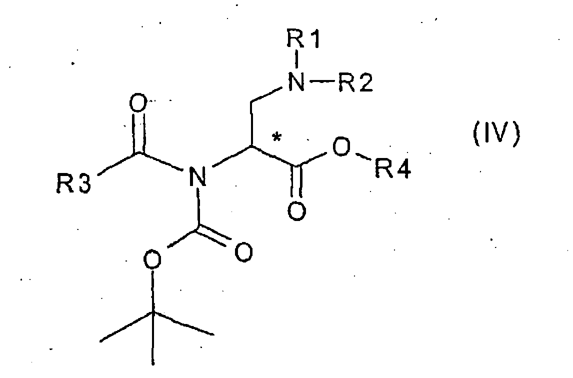 in which R1, R2, R3 and R4 are each as defined in formula I, and
c) then converting the resulting compound of the formula IV to the compound of the formula Ia 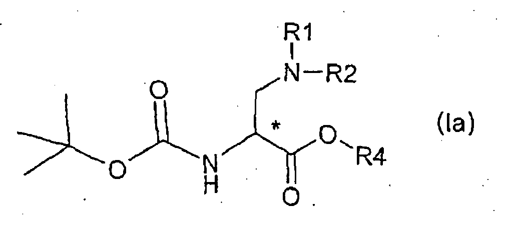 and
d) converting the resulting compound of the formula la to the compound of the formula III or salts thereof, in which R1, R2 and R4 are each as defined in formula I.

11. A process for obtaining the compound of the formula II as claimed in claim 1, which comprises reacting the compound of the formula VI 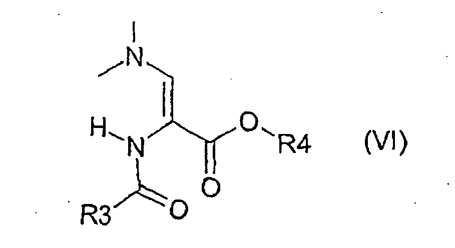 in which R3 and R4 are each as defined in formula II as claimed in claim 1
with an amine of the formula V
R1-N(H)-R2 (V)
in which R1 and R2 are each as defined in formula II as claimed in claim 1 to give the compound of the formula II.

12. A process for obtaining the compound of the formula IV as claimed in claim 2, which comprises
a) hydrogenating the compound of the formula II as claimed in claim 1,
in which R1, R2, R3 and R4 are each as defined in claim 2 in the presence of hydrogen and a catalyst and converting it to a compound of the formula I, and
b) reacting the resulting compound of the formula I with a tert-butyl dicarbonate and an acylation catalyst such as dimethylaminopyridine to give a compound of the formula IV 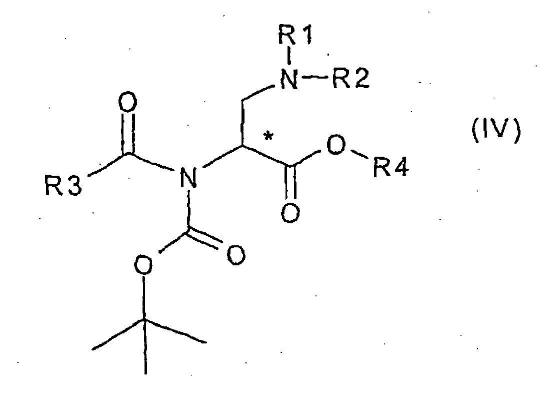
in which R1, R2, R3 and R4 are each as defined in claim 2.

## Revendications

1. Composés de formule II, 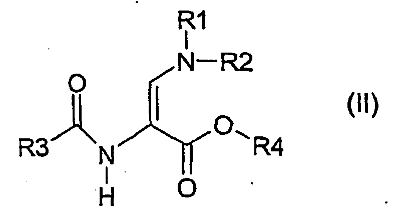 où R¹ et R² sont identiques ou différents et représentent, indépendamment l'un de l'autre
1) -(C₆-C₁₄)-aryle, où aryle est non substitué ou monosubstitué, disubstitué ou trisubstitué, indépendamment l'un de l'autre, par R¹¹,
où R¹¹ représente
a) F, Cl, I ou Br,
b) -(C₁-C₄)-alkyle,
c) -CN
d) -CF₃,
e) -OR⁵, où R⁵ représente un atome d'hydrogène ou - (C₁-C₄) -alkyle
f) -N(R⁵) -R⁶, où R⁵ et R⁶ représentent, indépendamment l'un de l'autre, un atome d'hydrogène ou -(C₁-C₄)-alkyle,
g) -C(O)-R⁵, où R⁵ représente un atome d'hydrogène ou -(C₁-C₄)-alkyle, ou
h) -S(O)ₓ-R⁵, où x signifie le nombre entier zéro, 1 ou 2 et R⁵ signifie un atome d'hydrogène ou -(C₁-C₄)-alkyle, ou
2) un hétérocycle de 4 à 15 chaînons, l'hétérocycle étant non substitué ou monosubstitué, disubstitué ou trisubstitué, indépendamment l'un de l'autre, par
-(C₁-C₅)-alkyle, -(C₁-C₅)-alcoxy, halogène, nitro, amino, trifluorométhyle, hydroxyle, hydroxy-(C₁-C₄)-alkyle, méthylènedioxy, éthylènedioxy, formyle, acétyle, cyano, hydroxycarbonyle, aminocarbonyle ou -(C₁-C₄)-alcoxycarbonyle,
R³ représente
1) -(C₆-C₁₄)-aryle, où aryle est non substitué ou monosubstitué, disubstitué ou trisubstitué, indépendamment l'un de l'autre, par -NO₂, -O-(C₁-C₄)-alkyle, F, Cl ou brome,
2) -O-C(CH₃)₃, ou
3) -O-CH(R⁷)-aryle, où aryle est non substitué ou monosubstitué, disubstitué ou trisubstitué, indépendamment l'un de l'autre, par -NO₂, -O-CH₃, F, Cl ou brome,
où R⁷ représente un atome d'hydrogène ou -(C₁-C₄)-alkyle,
R⁴ représente
1) un atome d'hydrogène
2) -(C₁-C₄)-alkyle ou
3) -CH(R⁸)-aryle,
où R⁸ signifie un atome d'hydrogène ou - (C₁-C₄)-alkyle.

2. Composé de formule IV, 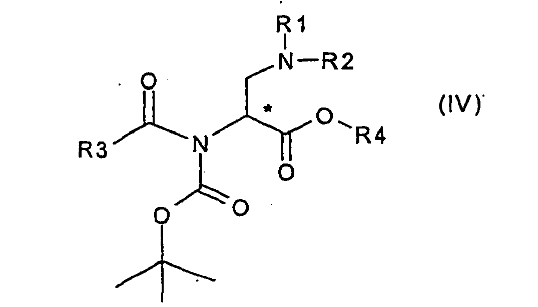 où R¹, R², R³ et R⁴ ont la même signification que dans la formule II selon la revendication 1.

3. Procédé pour la production du composé de formule I 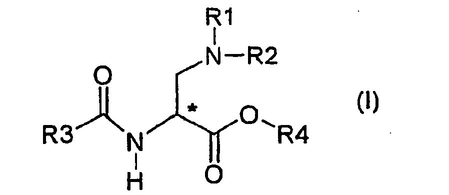 où R¹ représente
1) un atome d'hydrogène
2) -(C₁-C₄)-alkyle,
3) - (C₆-C₁₄) -aryle, où aryle est non substitué ou monosubstitué, disubstitué ou trisubstitué, indépendamment l'un de l'autre, par R¹¹, où R¹¹ représente
a) F, Cl, I ou Br,
b) -(C₁-C₄)-alkyle,
c) -CN
d) -CF₃,
e) -OR⁵, où R⁵ représente un atome d'hydrogène ou (C₁-C₄)-alkyle
f) -N(R⁵)-R⁶, où R⁵ et R⁶ représentent, indépendamment l'un de l'autre, un atome d'hydrogène ou -(C₁-C₄)-alkyle,
g) -C(O)-R⁵, où R⁵ représente un atome d'hydrogène ou -(C₁-C₄)-alkyle, ou
h) -S(O)ₓ-R⁵, où x signifie le nombre entier zéro, 1 ou 2 et R⁵ signifie un atome d'hydrogène ou -(C₁-C₄)-alkyle,
4) -CH(R⁷)-aryle, où aryle est non substitué ou monosubstitué, disubstitué ou trisubstitué, indépendamment l'un de l'autre, par -NO₂, -O-CH₃, F, C1 ou brome, où R⁷ signifie un atome d'hydrogène ou -(C₁-C₄)-alkyle, ou
5) un hétérocycle de 4 à 15 chaînons, l'hétérocycle étant non substitué ou monosubstitué, disubstitué ou trisubstitué, indépendamment l'un de l'autre, par -(C₁-C₅)-alkyle, -(C₁-C₅)-alcoxy, halogène, nitro,
amino, trifluorométhyle, hydroxyle, hydroxy-(C₁-C₄)-alkyle, méthylènedioxy, éthylènedioxy, formyle, acétyle, cyano, hydroxycarbonyle, aminocarbonyle ou -(C₁-C₄)-alcoxycarbonyle,
R² représente
1) -(C₆-C₁₄)-aryle, où aryle est non substitué ou monosubstitué, disubstitué ou trisubstitué, indépendamment l'un de l'autre, par R¹¹,
2) -CH(R⁷)-aryle, où aryle est non substitué ou monosubstitué, disubstitué ou trisubstitué, indépendamment l'un de l'autre, par -NO₂, -O-CH₃, F, Cl ou brome, où R⁷ signifie un atome d'hydrogène ou -(C₁-C₄)-alkyle, ou
3) un hétérocycle de 4 à 15 chaînons, l'hétérocycle étant non substitué ou monosubstitué, disubstitué ou trisubstitué, indépendamment l'un de l'autre, par -(C₁-C₅)-alkyle, - (C₁-C₅) -alcoxy, halogène, nitro, amino, trifluorométhyle, hydroxyle, hydroxy-(C₁-C₄)-alkyle, méthylènedioxy, éthylènedioxy, formyle, acétyle, cyano, hydroxycarbonyle, aminocarbonyle ou -(C₁-C₄)-alcoxycarbonyle,
R³ représente
1) un atome d'hydrogène
2) -(C₁-C₄)-alkyle,
3) -(C₆-C₁₄)-aryle, où aryle est non substitué ou monosubstitué, disubstitué ou trisubstitué, indépendamment l'un de l'autre, par -NO₂, -O-(C₁-C₄)-alkyle, F, Cl ou brome,
4) -O-C(CH₃)₃, ou
5) -O-CH(R⁷)-aryle, où aryle est non substitué ou monosubstitué, disubstitué ou trisubstitué, indépendamment l'un de l'autre, par -NO₂, -O-CH₃, F, Cl ou brome,
où R⁷ représente un atome d'hydrogène ou -(C₁-C₄)-alkyle,
R⁴ représente
1) un atome d'hydrogène
2) -(C₁-C₄)-alkyle ou
3) -CH (R⁸) -aryle ,
où R⁸ signifie un atome d'hydrogène ou -(C₁-C₄)-alkyle, **caractérisé en ce qu'**on hydrogène un composé de formule II 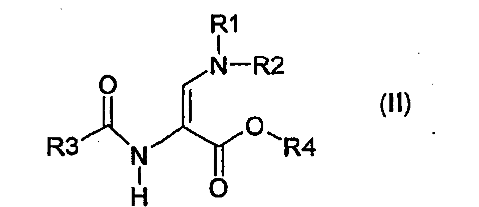 dans laquelle R¹, R², R³ et R⁴ ont la même signification que dans 1a formule I et le composé de formule II peut se trouver dans la configuration E ainsi que dans la configuration Z au niveau de la double liaison, en présence d'hydrogène et d'un catalyseur.

4. Procédé selon la revendication 3, **caractérisé en ce qu'**on obtient le composé de formule I,
où
R¹ représente phényle ou un atome d'hydrogène,
R² représente phényle, pyridyle ou thiazolyle, où phényle, pyridyle ou thiazolyle sont non substitués ou substitués par fluor ou chlore, et
R³ représente phényle ou -O-CH₂-phényle, et
R⁴ représente méthyle ou éthyle.

5. Procédé selon la revendication 3 ou 4,
**caractérisé en ce qu'**on utilise, comme catalyseur, des complexes du rhodium, du ruthénium ou de l'iridium ou des mélanges de ceux-ci.

6. Procédé selon l'une ou plusieurs des revendications 3 à 5, **caractérisé en ce que** le rapport molaire du catalyseur au composé de formule II est de 1:100 à 1:10 000.

7. Procédé selon l'une ou plusieurs des revendications 3 à 6, **caractérisé en ce que** l'hydrogénation est réalisée à une température de 10°C à 200°C et une pression d'hydrogène de 1 bar à 200 bars.

8. Procédé selon l'une ou plusieurs des revendications 3 à 7, **caractérisé en ce qu'**on utilise, comme solvant, de l'eau, des alcools tels que le méthanol, l'éthanol, le propanol ou l'isopropanol, des hydrocarbures aromatiques, tels que le toluène, des cétones telles que l'acétone, des hydrocarbures halogénés, tels que le dichlorométhane, les esters d'acides carboxyliques, tels que l'acétate d'éthyle et des éthers tels que le tétrahydrofuranne ou les mélanges de ceux-ci.

9. Procédé pour la production du composé de formule III, 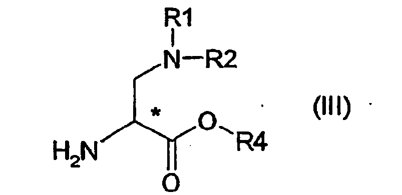 ou ses sels, dans laquelle R¹, R² et R⁴ ont la même signification que dans la formule I selon la revendication 3, **caractérisé en ce qu'**on
a) hydrogène le composé de formule II, 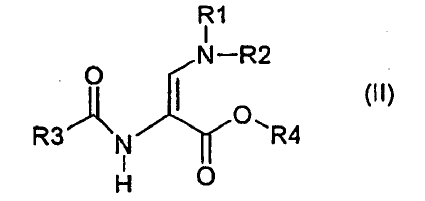 où R¹, R², R³ et R⁴ ont la même signification que dans la formule I,
en présence d'hydrogène et d'un catalyseur et transforme en un composé de formule I 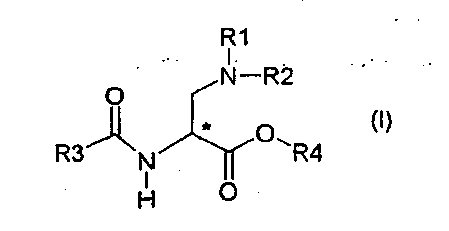 et
b) transforme le composé de formule I obtenu en un composé de formule III.

10. Procédé pour la production du composé de formule III selon la revendication 9, **caractérisé en ce qu'**on
a) hydrogène le composé de formule II,
où R¹, R², R³ et R⁴ ont la même signification que dans la formule I,
en présence d'hydrogène et d'un catalyseur et transforme en un composé de formule I,
b) transforme le composé obtenu de formule I avec du dicarbonate de tert-butyle et un catalyseur d'acylation tel que la N,N-diméthylaminopyridine en un composé de formule IV, 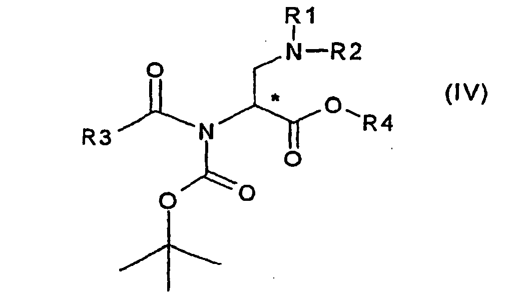 où R¹, R² R³ et R⁴ ont la signification comme dans la formule I, et
c) transforme ensuite le composé de formule IV obtenu en un composé de formule Ia 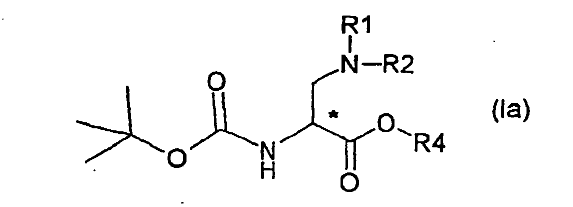 et
d) transforme le composé de formule Ia obtenu en composé de formule III ou ses sels, dans laquelle R¹, R², et R⁴ ont la signification comme dans la formule I.

11. Procédé pour la production du composé de formule II selon la revendication 1, **caractérisé en ce qu'**on transforme le composé de formule VI 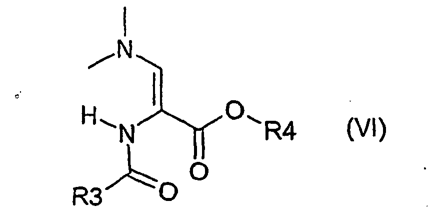 dans laquelle R³ et R⁴ ont la signification comme dans la formule II selon la revendication 1, avec une amine de formule V,
R¹-N (H)-R² (V)
dans laquelle R¹ et R² ont la signification comme dans la formule II selon la revendication 1, en composé de formule II.

12. Procédé pour la production du composé de formule IV selon la revendication 2, **caractérisé en ce qu'**on
a) hydrogène le composé de formule II selon la revendication 1,
dans laquelle R¹, R² R³ et R⁴ ont la même signification que dans la revendication 2 en présence d'hydrogène et d'un catalyseur et le transforme en un composé de formule I, et
b) transforme le composé de formule I obtenu avec du dicarbonate de tert-butyle et un catalyseur d'acylation tel que la diméthylaminopyridine en un composé de formule IV, 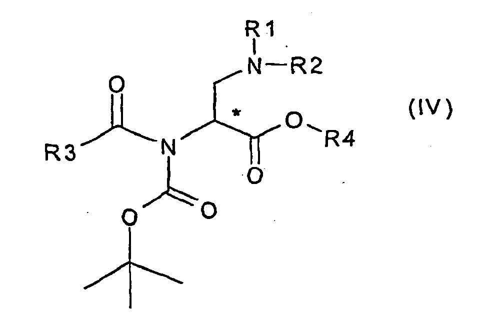
où R¹, R², R³ et R⁴ ont la même signification que dans la revendication 2.
